(19) **Europäisches Patentamt / European Patent Office / Office européen des brevets**

(11) **EP 3 047 309 B1**

(12) **EUROPEAN PATENT SPECIFICATION**

(45) Date of publication and mention
of the grant of the patent:
**05.03.2025 Bulletin 2025/10**

(21) Application number: **14771580.9**

(22) Date of filing: **22.09.2014**

(51) International Patent Classification (IPC):
**G01T 1/29** (2006.01)

(52) Cooperative Patent Classification (CPC):
**G01T 1/29; A61N 2005/1052; A61N 2005/1087**

(86) International application number:
**PCT/EP2014/070159**

(87) International publication number:
**WO 2015/040225 (26.03.2015 Gazette 2015/12)**

(54) **METHOD AND APPARATUS FOR THE RANGE CONTROL OF PARTICLE RADIATION OF A RADIATION DEVICE FOR RADIATION THERAPY**

**VERFAHREN UND VORRICHTUNG ZUR REICHWEITENREGELUNG DER TEILCHENSTRAHLUNG EINER BESTRAHLUNGSVORRICHTUNG FÜR EINE STRAHLENTHERAPIE**

**PROCÉDÉ ET APPAREIL POUR LA COMMANDE DE PLAGE D'UN RAYONNEMENT DE PARTICULES D'UN DISPOSITIF DE RAYONNEMENT POUR UNE RADIOTHÉRAPIE**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.09.2013 DE 102013218982**
**26.05.2014 EP 14169907**

(43) Date of publication of application:
**27.07.2016 Bulletin 2016/30**

(73) Proprietors:
- **Technische Universität Dresden**
  **01069 Dresden (DE)**
- **Helmholtz-Zentrum Dresden - Rossendorf e.V.**
  **01328 Dresden (DE)**
- **Ion Beam Applications S.A.**
  **1348 Louvain-la-Neuve (BE)**

(72) Inventors:
- **PAUSCH, Guntram**
  **01099 Dresden (DE)**
- **ENGHARDT, Wolfgang**
  **01454 Ullersdorf (DE)**
- **GOLNIK, Christian**
  **01099 Dresden (DE)**
- **JANSSENS, Guillaume**
  **1348 Ottignies-Louvain-la-Neuve (BE)**
- **PRIEELS, Damien**
  **1490 Court-Saint-Etienne (BE)**
- **SMEETS, Julien**
  **5004 Bouge (BE)**
- **VANDER STAPPEN, Francois**
  **1050 Bruxelles (BE)**
- **HUESO-GONZALEZ, Fernando**
  **01099 Dresden (DE)**

(74) Representative: **2s-ip Schramm Schneider Bertagnoll**
**Patent- und Rechtsanwälte Part mbB**
**Denninger Straße 169**
**81925 München (DE)**

(56) References cited:
**US-A1- 2011 057 110     US-A1- 2011 057 110**
**US-A1- 2012 025 076     US-A1- 2012 025 076**

- **TESTA E ET AL: "Dose profile monitoring with carbon ions by means of prompt-gamma measurements", NUCLEAR INSTRUMENTS & METHODS IN PHYSICS RESEARCH. SECTION B: BEAM INTERACTIONS WITH MATERIALS AND ATOMS, ELSEVIER BV, NL, vol. 267, no. 6, 1 March 2009 (2009-03-01), pages 993 - 996, XP026067102, ISSN: 0168-583X, [retrieved on 20090211], DOI: 10.1016/J.NIMB.2009.02.031**

- MAURO TESTA: "Charged particle therapy, ion range verification, prompt radiation", 14 October 2010 (2010-10-14), XP055561502, Retrieved from the Internet <URL:https://tel.archives-ouvertes.fr/tel-00556628/document> [retrieved on 20190226]
- TESTA E ET AL: "Dose profile monitoring with carbon ions by means of prompt-gamma measurements", NUCLEAR INSTRUMENTS & METHODS IN PHYSICS RESEARCH, SECTION - B:BEAM INTERACTIONS WITH MATERIALS AND ATOMS, ELSEVIER, AMSTERDAM, NL, vol. 267, no. 6, 1 March 2009 (2009-03-01), pages 993 - 996, XP026067102, ISSN: 0168-583X, [retrieved on 20090211], DOI: 10.1016/J.NIMB.2009.02.031

# EP 3 047 309 B1

## Description

The invention relates to methods and apparatuses for the verification of the range of a charged hadronic particle of a radiation device for radiation therapy by measuring prompt-gamma radiation being produced by the charged hadronic particle radiation when passing the tissue of an object to be radiated with at least one detector, being able to detect single gamma particles of the prompt gamma radiation and at least one analyzer.

High energetic beams of protons or heavy ions, summarized as particle radiation, do release their energy primarily at the end of the stopping distance when entering tissue. The dependency of the stopping power from the covered distance with the typical maximum, the so-called Bragg-Peak at the end of the path does allow the selective destruction of more bad tissue with minimal side effects to the surrounding healthy tissue. Therefore, particle radiation is especially suitable for the therapy of tumors. Nevertheless, the position of the particle radiation within the tissue, especially its termination point, the so-called distal edge, needs to be monitored carefully in order to avoid an unwanted destruction of healthy tissue. This monitoring should occur in real-time already during therapy, so that a radiation treatment with a defective beam range can be corrected or terminated in time. Generally, it is assumed that such a monitoring of a therapy can be realized best by measuring prompt gamma radiation, being produced by the particle beam when passing the tissue. This gamma radiation does pass the body nearly undisturbed and can be detected with suitable detectors near the patient. The emission points of the prompt gamma radiation are distributed along the particle trails and strongly correlated with the dose distribution, deposed by the particle beam in the tissue. According to the present state of the art it is therefore assumed that only an image guided measurement of that prompt gamma radiation does allow for the desired monitoring of the therapy in real time.

In order to realize such an image guided measurement of prompt gamma radiation, two general concepts are followed by the state of the art. Those are, as described inter alia in the textbook "Proton Therapy Physics" by H. Paganetti (Editor), CRC Press, Taylor & Francis Group, LLC, 2012, pages 512-514, systems with passive collimators and a detector with spatial resolution on the one hand and systems without collimators being operated under the Compton camera principle on the other hand.

Compton cameras do work by utilizing the principle of Compton imaging. Hereby, a gamma particle is scattered within a scattering detector electron (Compton scattering). A recoil electron does produce a signal within the scattering detector, from which the energy loss of the gamma particle can be determined. The scattered gamma particle may leave the scattering detector and may, with a certain probability, generate signals in one or more further detectors in case it is absorbed therein completely or scattered again. From the energy losses in the single detectors, conclusions regarding the direction of the incoming gamma particle before the occurrence of the first scattering can be obtained when the places of interaction are known. The evaluation is especially simple and exact when the scattered gamma particle is fully absorbed already when it first interacts with one of the further detectors. The measurement of a multitude of gamma particles does allow the reconstruction of the source distribution of the gamma radiation when using suitable algorithms. Consequently, Compton cameras do comprise at least two detectors with both spatial and energy resolution, mostly arranged in different planes. It can be derived from the principle of the Compton imaging that the spatial resolution of a system and therefore the accuracy of the therapy monitoring is dependent from both, the spatial as well as the energy resolution of the detectors used. A sufficient resolution in space and energy can be obtained only with very high quality detectors and a related complex electronics. Therefore, Compton cameras are very complex, expensive and difficult to realize.

Systems using a collimator do have a much more simple setup when compared with Compton cameras. They consist of one or more collimators and a detector plane with a spatial resolution, detecting the gamma radiation behind the collimator. When evaluating the shading of the collimator, absorbing the prompt gamma radiation at least in part, information about the source points of the radiation can be obtained. This is derived solely from the spatial distribution of the gamma radiation in the detector plane so that an exact measurement of the energy is not necessary here. This measurement generally does require substantially lower demands to the detectors, which may then be selected from a broad line up of available materials, which is also true for the electronic. Nevertheless, very massive collimators are necessary as they must weaken the gamma radiation with energies in a MeV range so heavily that there is a sufficient shading. The thickness of the collimator is an obstacle for the construction of coded collimators or masks, which may be used in nuclear medicine for the making of two or three dimensional pictures of the gamma source distribution also.

From US2011/0057110A1 it is known to make use of a multitude of detectors, each detector being collimated in order to just monitoring a small section of the target. In addition, it is proposed to discriminate against neutron induced back-ground radiation by measuring the time of flight, thereby suppressing the slower neutrons. This allows to omit a bulky neutron shielding.

US2012/0025076A1 proposes the use of a second detector with a multitude of independent detector plates in order to determine the angle under which the de-tected gamma rays enter the detector. Knowing this angle allows the system to determine the area of origin within the target.

Testa et al, Nucl.Inst.Meth. B 267 (2009) 993, discloses the simultaneous discrimination of energy and time of flight information, thus allowing for a better suppression of background radiation.

Testa, "Charged particle therapy, ion range verification, prompt radiation", Physics Universite Claude Bernard, 2010, Chapter 5, does also discuss the spatial distribution of prompt gamma rays along the particle beam path. It is proposed to discriminate against gamma and neutron background by counting prompt gamma radiation only at various positions along the beam axis, thereby using a lead collimated detector with a BaF2 scintillator.

Systems known so far are generally aimed at the image guided measurement of prompt gamma radiation. An image guided measurement is not possible with a simple, non segmented detector, as it, according to the state of the art, does require the detection of single prompt gamma particles with a spatial resolution in any case. Respective systems do therefore consist either of a multitude of a smaller single detectors or of segmented detectors or detectors with a spatial resolution, which do allow for a measurement of the place of interaction within the detector. Common to all systems is a measurement of the timing of the interaction within the detectors also. On the one hand, this timing information is used to identify the coincident response of detectors (coincidences). This is mandatory in Compton cameras. On the other hand, the measurement of the timing difference between the detection of a gamma particle and a periodic reference signal for particle bunches out of the pulsed radiation device do allow for a clear separation between the prompt gamma radiation, being correlated in time to the particle bunch, and the correlated back-ground, disturbing and deteriorating the measurement. Thereby, the peak to back-ground ratio can be improved and, as a consequence, the quality of the image also. The timing resolution of the detectors used must be within a range of at least one or a few nanoseconds. This timing is used only for a classification of the events, in which a gamma particle has been detected in the image guided detector system. It is auxiliary factor for the selection of the events, being suitable for the imaging and the respective devices. Structure and location of the so-called prompt peak within the time distribution are not analyzed further.

It is common to known systems that, according to the measurement principle, only a part of the impinging gamma particles can be used for the imaging process. Systems using a collimator cannot use the gamma particles hitting the collimator, as those produce unwanted background at the maximum. Compton cameras do only make correct use of those gamma particles, which do see exactly one Compton scattering within the scattering detector and which are consequently absorbed in the absorber. Those selection criteria do reduce the number of usable detector hits against the number of gamma particles, which do hit the detection system as a whole, usually by at least one order to magnitude. One can act on the assumption that, considering the constraints during therapy, a measurement time of a few seconds will be not sufficient to produce an image, allowing for a range control with sufficient precision. During the short radiation exposure in therapy, such images with a sufficient statistic cannot be obtained fast enough in order to actively influence the process of the radiation fraction.

Detectors for detecting single gamma particles are, inter alia, known scintillation detectors, Cherenkov detectors or so-called Resistive Plate Chambers (RPC).

It is known that a scintillation detector does consist of a scintillator and a photo detector. The scintillator may be a fast scintillator made from inorganic materials or a fast organic scintillator made from plastics or an organic liquid scintillator. The fast scintillators do make sure that the scintillation detector will provide a sufficiently good timing resolution.

For example, a known Cherenkov detector as published in the textbook "Radiation Detection and Measurement" by G.F. Knoll, John Wiley & Sons, Inc., 4th Edition, 2010, pages 733-735, may consist of a photo detector coupled to a transparent medium. The transparent medium is preferably solid or liquid, a known scintillating inorganic crystal, glass, water, a non-scintillating organic material or a non-scintillating organic material with wavelength shifting additives for the conversion of short wave photons to photons with a longer wavelength, which can be detected by the photo detector with higher efficiency. It is possible to obtain an even better timing resolution with a Cherenkov detector as with fast scintillation detectors.

A Resistive Plate Chamber (RPC), known from the textbook "Radiation Detection and Measurement" by G.F. Knoll, John Wiley & Sons, Inc., 4th edition, 2010, pages 201-203, does consist of at least two plates with suitable conductibility, being separated by a gap, filled with gas, which are between electrodes, connected to a high voltage. Such kind of detector do show an especially good timing resolution.

The invention as described in patent claims 1 to 12 is based on the problem to substantially simplify, reduce the complexity, increase the robustness and to substantially reduce the measurement time being necessary for verification methods and apparatuses for the verification of particle therapies.

This problem is solved by the features described in independent claims 1 and 7 and further specified in claims 2 to 6 and 8 to 12. The invention relates to a method according to claim 1, and to an apparatus according to claim 7.

The methods and devices for the control of the range of a particle radiation in a radiation device for radiation therapy by the measurement of prompt gamma radiation stand out especially by the simple, complexity reduced and robust realization as well by the strongly reduced measurement time.

In that way a statement regarding the conformity of the therapy to be monitored with this device with the radiation plan, mainly based on this time distribution, is possible.

During the treatment of patients with particle beams, gamma radiation is created also, which cannot be allocated to the prompt gamma radiation as created by the therapy beam in the tissue. This radiation underground does not contribute to the effect to be measured, but may disturb or falsify the time distribution, which is used for the monitoring of the therapy. Characteristic for the prompt gamma radiation is its high energy and the preferred emission of discrete energies (emission lines) in a range between 1 MeV to 8 MeV. It is therefore advantageous not to make use of all events measured by the detector, but to filter those events according to some criteria, which prefer prompt gamma radiation. Such a selection (filtering) may be made for example by the additional analysis of the detector signals with respect to their amplitude. With an amplitude filter, it is possible to filter events in the analyzer, which depose energies between 2 MeV and 8 MeV or especially preferred between 3 MeV and 5 MeV or between 5 MeV and 7 MeV within the detector. The time distribution is then obtained only from the selected (filtered) events and does contain, once a suitable selection of filter conditions has been made, mainly events, which relate to the detection of prompt gamma radiation with a reduced background compared to the time distribution of the unfiltered events. Other criteria may be based on a selection of the signal form. This is, for example, an advantage when the gamma radiation is detected with liquid organic scintillators, which do also detect neutron radiation. By applying several independent filters, several time distributions can be created in parallel and analyzed separately.

In principle, it is possible to select the events, being used to create the time distribution, arbitrarily or regularly from the total number of all events without distorting the measurement effect. This may be useful, for example, when the count rate in the detector is so high that it cannot be mastered by the analyzer in real time. In such a case it is nevertheless possible also to use several analyzers in parallel, which may be connected for example with several separate (smaller and thinner) detectors with a reduced effectivity and therefore a reduced count rate.

The realization of the method and the event is therefore based upon a new principle for the range control of particle beams in tumor therapy. Thereby, as in other and known concepts, the signature of prompt gamma radiation is used. Contrary to the known concepts, which are based on a measurement of the places of interaction with a resolution being as good as possible and which are, in the case of Compton cameras, based on the energies of interaction of prompt gamma radiation in specified detectors of a respective device, the method and the device according to the present invention do mainly evaluate the interaction times of respective gamma particles in the detector only. Specific advantages are that such a device according to the invention can be realized already with one single, simple constructed and cheap detector as well as with a, compared to other concepts, strongly reduced electronic effort, and that the result of the therapy monitoring can be provided in a substantially reduced measurement time, compared to other concepts. Using at least two detectors under different angles with respect to the direction of the beam does allow an even more sophisticated evaluation as the position of those detectors can be considered in addition to the measured time distributions.

The method and the device make use of the fact that the high energy particles, being used for therapy, do move very quickly, but do nevertheless need a limited time until they reach their target volume, which can be measured in principle. Particles, which do have a larger range in the object to be radiated as planned, need to travel a longer way until they reach the area of maximum energy loss (Bragg-Peak), therefore arriving there later and therefore causing the emission of prompt gamma radiation later in average than the reference particles according to the plan. Vice versa, the prompt gamma radiation is emitted earlier on average in case of a shorter range. Simple estimations show for example that protons with 150 MeV do need about 1 to 2 ns (nanoseconds) from the entrance into the body until they slowed down completely and that a difference in distance of 1 cm does cause a timing difference in the area of about 100 ps (picoseconds). Such timing differences can generally be measured with state of the art detectors. Those detectors do not need to have a spatial resolution and a measurement of the energy is not mandatory also. Even a single detector in a suitable setup is sufficient to detect shifts in range of the therapy beam on the basis of a timing measurement. Prerequisite is that there is an exact timing reference for the passing of the therapy particles through a well-defined reference plane perpendicular to the direction of the beam, which may nevertheless being choosen arbitrarily. As usual radiation devices are high frequency accelerators, which deliver short beam bunches, being exactly synchronized with the acceleration frequency, this high frequency may be used as a timing reference. Nevertheless, it is also possible to derive the timing reference from specific detectors, which are passed by the beam particles and which do deliver a correlated timing signal for each beam packet or even for each single beam particle. An example for such a detector is a so-called beam hodoskop.

By the method and the device, the statistical distribution of timing and differences between the detection of prompt gamma particle in the detector and a reference signal is determined, which provides the time at which the therapy particle or the particle bunch, which has caused the gamma emission, has passed the reference plane. When using two or more detectors, it is also possible to determine the relative timing information between those detectors and to consider their position in space in addition. The measured distribution is identified as time distribution in the following. It can be determined either from all events or from a selection of events. With the method and the device according to the invention, movements in range compared to the planned situation are determined mainly from this time distribution. This may occur for example by determining suitable integral parameters from the measured time distribution and comparing them with a time distribution of a reference. Therewith, a sensibility for changes in range in the order of a few millimeters can be achieved.

Examples for integral parameters may be

- the position of the center of the distribution,

- the position of the maximum of a function adapted to the distribution, or
- the width of a distribution, for example expressed by the variance or the dispersion of the distribution or by the full width half maximum of a function being adapted to the distribution.

The calculation of integral parameters of a single spectrum and thereby the monitoring of the range may, other than in complex calculations for the reconstruction of images of other methods, occur in parts of a second, that is in real time parallel to the measurement. As the measurement principle is based on a "straight forward measurement" without limiting requirements like coincidence or collimators, the number of detector events per therapy particle which can be evaluated, is, referred to the solid angle covered by the detector, much higher than in all known methods with an image guided measurement of prompt gamma radiation on the basis of a measurement of the spatial distribution. Time distributions with sufficient statistics for a range monitoring can be measured with standard detectors under conditions of a therapy within a few seconds or may be even in one second, so that possible faults in range can be detected and corrected at the begin of the therapy (fraction) already. The measurement method materialized in the apparatus can be realized easily in a clinic and can be integrated well in clinical processes.

According to an improvement, the analyzer does select events, for which the time distribution is created according to predetermined criteria to the signal of the detector. Criteria are, according to a further improvement, the magnitude, the amplitude and/or the form of the signal of the detector.

According to the invention, the analyzer is an analyzer, analyzing the time distribution, thereby deriving information about the range of the particle beam, so that a statement regarding the conformity with the therapy to be monitored by the device with the radiation plan is given in real time, so that it is possible to either continue the radiation, interrupt the radiation or modify the radiation by changing the setup of the radiation device. As a consequence, the information regarding possible deviations of the therapy from the radiation plan are available faster, so that real time therapy monitoring becomes possible. Already during the running therapy, preferably no later than 10 seconds, especially no longer than 2 seconds after the begin of the therapy, it can be interrupted or corrected.

The measure for the range is, according to a further improvement, preferably derived from the time distribution. A statement regarding the conformity or the actual range of the particle beam is, according to an even further improvement, derived from an integral parameter of the time distribution. For example, at least one statistical moment of the time distribution does, according to a further improvement, define the conformity or the range of the particle radiation. Those may be for example the center of gravity and/or the variance of the time distribution.

At least one fitting parameter, described by the given and approachable or adaptable function to the time distribution does determine the conformity or the range of the particle radiation. That can be for example the position, the width and/or the asymmetry of a peak of the time distribution, which is adapted by a suitable function, for example a Gauss-function or a Pearson-function.

It is a specific advantage if, before the treatment begins, there is a reference target at the place of the patient in the particle beam, which is used to investigate at least one time distribution as a reference so that a comparison of the time distributions between the reference and the time distributions during the radiation of a patient can provide a statement with respect to the range of the particle radiation.

In a more specific setup, the radiation device does comprise at least a second gamma detector for detecting prompt gammas emitted from said target, the second detector located with respect to the first detector such that the first and the second detector are detecting prompt gammas emitted from different angles with respect to said beam direction. The device is therefore able to obtain a first time distribution for the first gamma detector and obtaining a second time distribution for the second gamma detector, thereby determining from said first time distribution a first time width $\Delta T1$, determining from said second time distribution a second time width $\Delta T2$, and determining a photon travel shift $\Delta DP$, defined as a distance travelled by a photon in the time interval equal to the difference between the first and the second time width multiplied by the speed of light c as

$$\Delta DP = (\Delta T2 - \Delta T1)*c.$$

Finally, the range of the particle beam is determined by correlating said photon travel shift $\Delta DP$ with the difference in detector location between the first and the second detector.

One method to determine said penetration depth comprises a step of calculating a distance R in the target by solving the equation

$$\Delta DP = SQRT(R*R + d1*d1 - 2*R*d1*cos(\alpha)) -$$

$$SQRT(R*R + d2*d2 - 2*R*d2*cos(\beta)) - (d1-d2),$$

whereby ΔDP is the said photon travel shift, d1 is the distance from the first detector to the entrance point, the entrance point being defined as the point where the energetic particle beam enters the target, d2 is the distance from the second detector to the entrance point, α is the angle between the beam direction and the direction going from the entrance point towards the first detector, β is the angle between the beam direction and the direction going from the entrance point towards the second detector.

Preferably, said second gamma detector is located with respect to said first detector such that the first and the second detector are detecting prompt gammas emitted at angles with respect to the beam direction that differ by at least 20°. For example, said first gamma detector can be located for measuring prompt gammas emitted at an angle equal or larger than 100° with respect to the beam direction and said second gamma detector can be located for measuring prompt gammas emitted at an angle equal or smaller than 80° with respect to the beam direction accordingly.

According to a further improvement, it is advantageous if the detector, being able to detect single gamma particles, is a scintillation detector, comprising at least one scintillator and at least one photo detector, a Cherenkov detector, comprising at least one photo detector, coupled to a transparent medium, or a Resistive Plate Chamber (RPC). With the mentioned detectors, a resolution in time, being sufficient for the purpose of the measurement, is achievable.

In order to being able to conduct the method steps described above, it is advantageous if the claimed apparatus further comprises a second gamma detector, configured for detecting prompt gammas emitted from said target, being located with respect to said first detector such that the first and the second detector are detecting prompt gamma particles emitted from different angles with respect to the beam direction, the analyzer calculating the time differences $\Delta t1$ and $\Delta t2$ for both detectors so as to obtain time distributions for both detectors, further comprising an analyzer comprising an algorithm configured for determining from said first time difference $\Delta t1$ a first time width $\Delta T1$ and from said second time difference $\Delta t2$ a second time width $\Delta T2$, further determining a photon travel shift $\Delta DP$ defined as a distance travelled by a photon in the time interval equal to the difference between the first and the second time width:

$$\Delta DP = (\Delta T2 - \Delta T1) * c$$

In this context, c is equal to the speed of light, determining a particle beam penetration depth by correlating said photon travel shift $\Delta DP$ with the difference in detector location between the first and second detector.

This apparatus does preferably comprise an analyzer with computing means configured for calculating a distance R in the target by solving the equation

$$\Delta DP = SQRT(R*R + d1*d1 - 2*R*d1*\cos(\alpha)) - SQRT(R*R + d2*d2 - 2*R*d2*\cos(\beta)) - (d1-d2),$$

In this context $\Delta DP$ is the said photon travel shift, d1 is the distance from the first detector to the entrance point, the entrance point being defined as the point where the energetic particle beam enters the target, d2 is the distance from the second detector to the entrance point, α is the angle between the beam direction and the direction going from the entrance point towards the first detector, and β is the angle between the beam direction and the direction going from the entrance point towards the second detector. Preferably, said second gamma detector is located with respect to said first gamma detector so that the first and the second gamma detector are detecting prompt gammas emitted at angles with respect to the beam direction that differ by at least 20°. In a most preferred setup, said first gamma detector is located for measuring prompt gammas emitted at an angle equal or larger than 100° with respect to the beam direction at the beam exit and said second gamma detector is located for measuring prompt gammas emitted at an angle equal or smaller than 80° with respect to the beam direction at the beam exit.

An embodiment of the invention is described in a general form in the figures and is described closer in the following.

It shows:

Fig. 1    a device for the monitoring of the range of a particle radiation of a radiation device for the use in radiation therapy,
Fig. 2    a device with a radiation device comprising a particle detector,
Fig. 3    a device during a therapy,
Fig. 4    a device during calibration,
Fig. 5    a device with several detectors,
Fig. 6    a schematic setup of a device using two detectors in relation to the beam source,
Fig. 7    an example prompt gamma emission and detection profiles,
Fig. 8    an example prompt gamma emission and detection profiles whereby a cavity is present in the target,

Fig. 9      differences in the width $\Delta T2$-$\Delta T1$ of timing profiles according to the invention,

Fig. 10     differences in sensitivity between a single and a two-detector setup, and

Fig. 11     shows differences in width $\Delta T2$-$\Delta T1$ for various setup configurations.

Method and apparatuses for the monitoring of the range of a particle radiation of a radiation device for use in radiation therapy according to the invention are further explained.

A device for the monitoring of the range of the particle radiation of a radiation device 1 for radiation therapy consists mainly of a radiation device 1, at least one detector 2, being able to detect single gamma particles, and at least one analyzer 7.

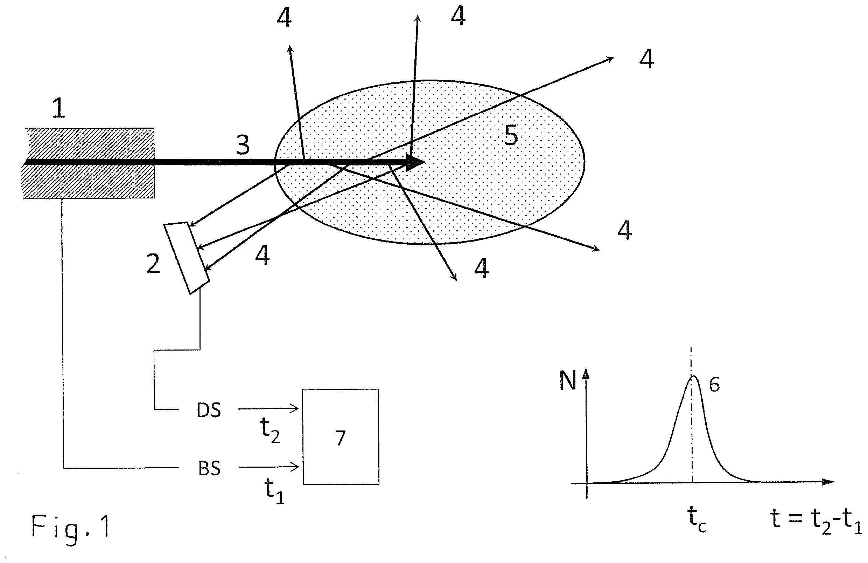

Fig. 1 shows a device for the monitoring of the range of a particle radiation of a radiation device 1 for the use in radiation therapy in a general setup.

Fig. 1 shows schematically a device in its simplest setup. The radiation device 1 sends a pulsed particle beam 3 into the patient 5. Beside this, the radiation device 1 delivers a time reference signal, the bunch signal BS, which is exactly correlated with the beam bunches. The particle beam 3 generates prompt gamma radiation 4 within the patient 5. A part of this radiation 4 strikes detector 2, which preferably is to be arranged in backward direction, seen from the particle beam 3. This detector 2 delivers a detector signal DS when a gamma particle 4 is detected. The analyzer 7 analyzes the detector signal DS and the correlated bunch signal BS, deriving the points in time $t_2$ and $t_1$, which correspond to the striking of the gamma particle 4 in detector 2 as well as to the point in time at which the initiating particle or particle bunch has passed though a reference plane perpendicular to the particle beam 3, calculates the time difference $t = t_2 - t_1$ and creates the time distributions 6 of all measured time differences. Because of the limited flight path of the particles of the particle beam 3 from the reference plane to the patient 5 as well as within the patient 5 itself and the limited time of flight of the gamma particles 4 from their place of emission alongside the particle path to detector 2, this distribution has a limited dimension in time. Characteristic integral parameters of the time distribution 6, like the average (center of gravity) or the mostly occurring time difference (maximum) or the width (scattering) of the time distribution 6, here symbolically depicted with $t_c$, can be determined unambiguously and very exact when there is sufficient statistics. Those values $t_c$ do characterize, according to the argumentation above, the range of the particle beam 3. They change if the range changes and are therefore used to determine the range or to monitor the conformity of the treatment with the treatment plan. The functional dependence of those parameters $t_c$ from the range can be determined from the reference distributions, which were either measured before under monitored setups or with the aid of respective simulation software in the framework of the radiation planning.

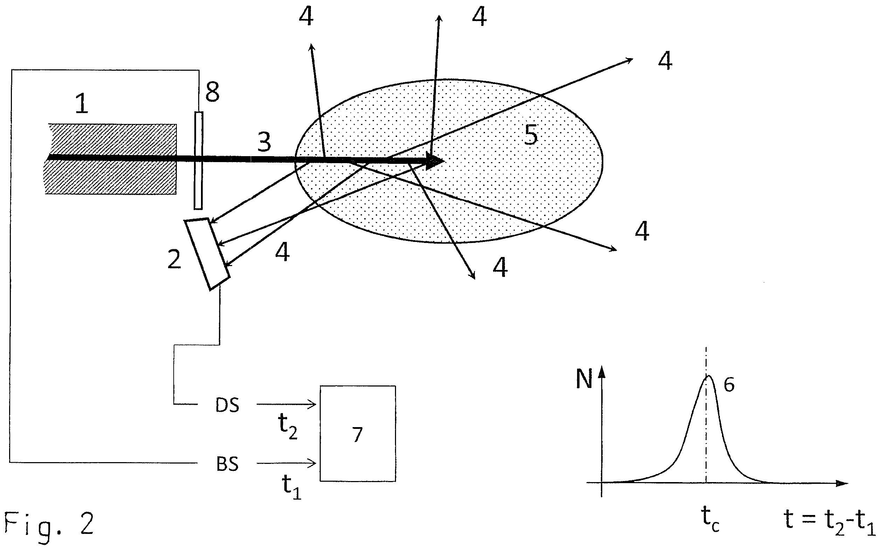

Fig. 2 shows an apparatus with a radiation device 1, supplemented by a particle detector 8 in a general setup.

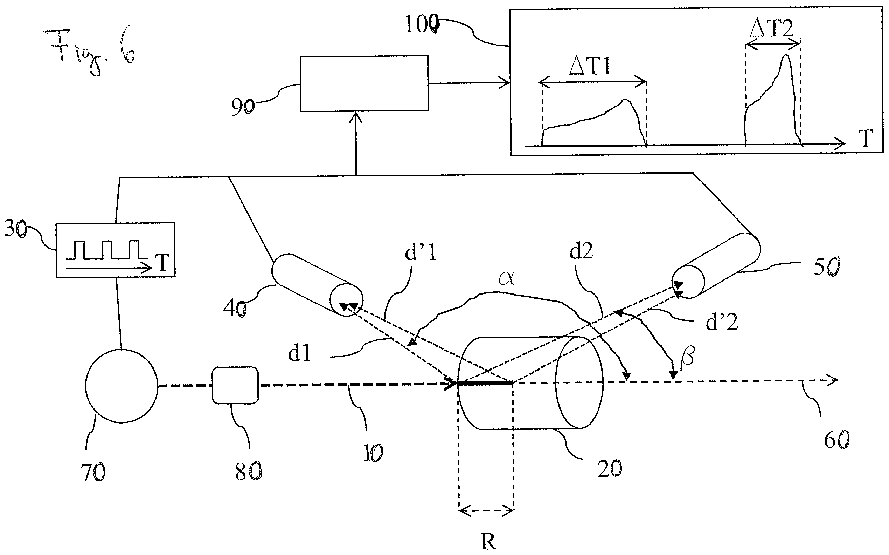

Fig. 2 shows a device, where the time reference signal for the particle packet BS is not delivered by the radiation device 1, but by a separate particle detector 8. It is arranged within the particle beam 3, usually perpendicular to the particle beam 3, and does create a signal BS at every passing of a single particle or a particle packet of the radiation device 1, which is correlated in time with the passing of the particle through the particle detector 8. The creation of the time distribution 6 does then occur in the manner as described in the context of the setup of Fig. 6.

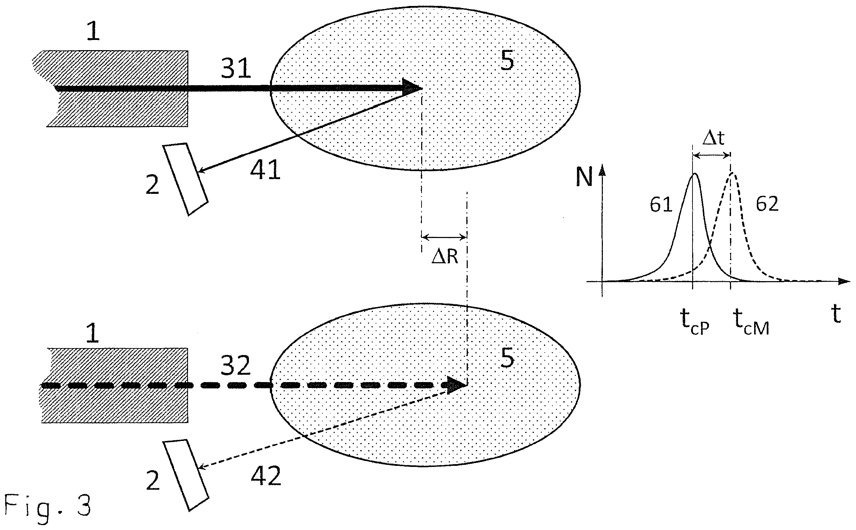

Fig. 3 shows a device during a therapy in a general setup.

Fig. 3 shows a schematic situation, in where the real range of a particle beam 32 within the patient is larger than the forecasted range during the planning of the radiation (particle beam 31). Each beam packet of the particle beam 32 therefore needs a longer time than planned until it is stopped completely. The prompt gamma particles 4 are emitted proportionally later in average. In addition, the mean distance which those gamma particles 4 have to travel to detector 2 is increased. As a consequence, the measured distribution 62 of the time differences is shifted against the planned situation by the amount $\Delta t$, whereby the form of the distribution, especially its width, does change in certain limits also. Even with respect to the change in its form, the shift can be quantified, for example by a determination and a comparison of the centers of gravity of both distributions. The shift of the characteristic point $t_{cM}$ of the measured distribution against a respective point $t_{cP}$ of a distribution according to the planning is therefore a measure for the real change in range.

In case the timing resolution of detector 2 is sufficiently good, it is also possible to obtain information about the range from the width of the distribution, for example characterized by the second statistical moment or the statistic variance of the time distribution 6. This is a consequence from the fact that the emission of prompt gamma radiation mainly starts when the particle bunch enters the patient and ends when the particle bunch has been completely stopped. As the particle bunch 32 does need more time for the slowdown process than the particle bunch 31, the respective time distribution 62 is wider than the time distribution 61.

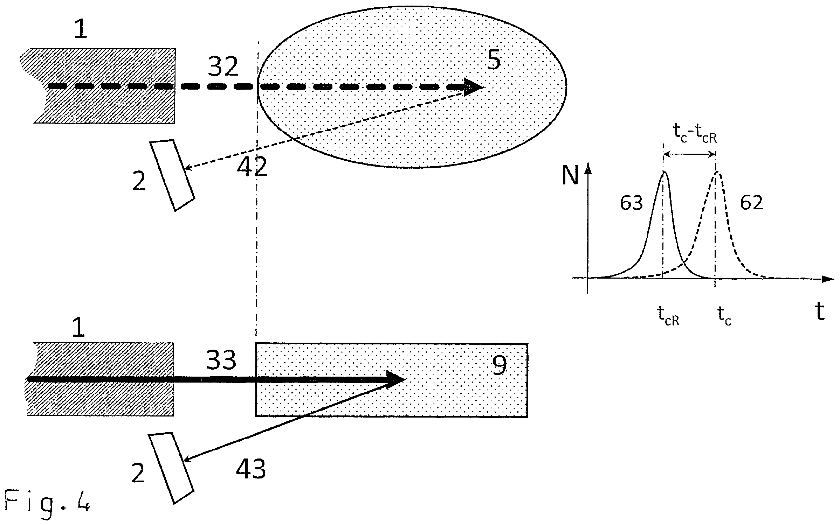

Fig. 4 shows a device during the calibration in a general setup.

This calibration does solve a problem, resulting from the fact that the times $t_1$ and $t_2$ being derived from the signals BS and DS, are afflicted with an offset, which is hardly to quantify. Those offsets result from the properties of the detectors 2 being used, from the length of the used signal cables to analyzer 7, from actual setups of the accelerator device including the beam line to the patient 5 and other parameters. The determination of an absolute value $t_{cM}$, which could be compared to an absolute reference value $t_{cP}$ according to the presentation of fig. 3, does nevertheless require the knowledge of those offsets or at least their difference in some cases, for example when the center of gravity or the position of the maximum of the distribution is used as a critical parameter $t_c$.

The determination of the offset or its difference can be omitted, when

- both, the radiation of a patient 5 and the radiation of a reference target 9 are modelled with the same beam parameters in the radiation planning, whereby the radiation planning does also include the modelling of respective time distributions 6,
- the characteristic parameters $t_{cR}$ and $t_c$ and their difference $\Delta t_P = t_{cR} - t_c$ as "planned parameters", are determined from modelled time distributions 63 (for the reference target 9) and 62 (for the patient 5) according to the radiation planning,
- a time distribution 63 is measured with a reference target 9 with the same beam parameters as they are planned for the radiation exposure of the patient 5 before the radiation exposure of the patient 5 starts,
- the patient 5 is being exposed afterwards and a time distribution 62 is measured during the exposure,
- the characteristic parameters $t_{cR}$ and $t_c$ and their difference $\Delta t_M = t_{cR} - t_c$ are determined as "measured parameters" from the measured time distributions 63 (with reference target 9) and 62 (with patient 5), and
- information about the conformity of the exposure with the respective exposure planning or about the range of the particle beam 3 during the exposure is derived from a comparison of the measured difference $\Delta t_M$ with the planned difference $\Delta t_P$.

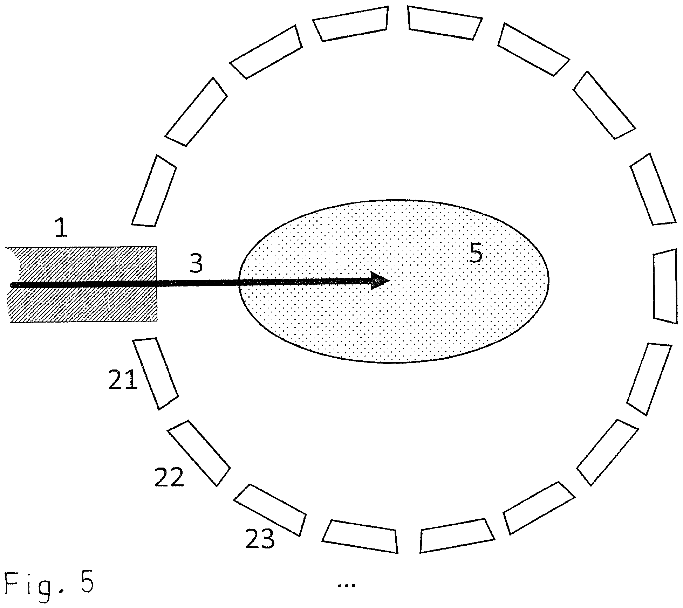

Fig. 5 shows a setup with several detectors in a general presentation.

The use of several detectors instead of a single detector can be advantageous for several reasons.

At first, the accuracy of the method can be improved by increasing the number of detectors 2, 21, 22, 23 ... Using one or more analyzers 7 allows for the evaluation of a respective larger number of time distributions 6 with an overall larger number of events so that the statistical uncertainty of the conclusions being derived from the time distribution 6 can be reduced.

At second, the use of several detectors can reduce the time until a predetermined number of events is reached, corresponding to a predetermined statistic certainty of conclusions about the conformity of the treatment with the exposure planning. In this way, the time until a decision about the continuation, interruption or correction of a running exposure can be reduced.

At third, some radiation devices 1 do deliver beam packets not continuously, but in larger intervals, but at the same time with very high intensity. Examples for such radiation devices 1 are synchrocyclotrons or laser driven particle accelerators, being still under development. In such a case, the number of events per detector 2 can be very high in their intensity maximum when detector 2 is dimensioned in a way that it does detect the necessary average number of events per second, necessary for therapy monitoring. The load per detector 2 can be reduced, if one makes use of several smaller or thinner detectors 2. The average number of events (detected prompt gamma particles) per second, being necessary for therapy monitoring, is so distributed among many single detectors or detector segments, so that the load per detector 2 within the intensity maximum is acceptable and the measurement is not falsified by pulse pile up and similar effects.

Finally, the use of two or more detectors does allow for a more sophisticated evaluation of the data as will be described in the following.

A radiation device in combination with the inventive apparatus for monitoring the range of the particle radiation, in the following further specified as a particle beam system, is schematically illustrated in Fig. 6. The particle beam system according to the invention is comprising a particle beam source 70 for delivering energetic particles or bunches of energetic particles. Those energetic particles or bunches of energetic particles are forming an energetic particle beam 10. The particle beam source can for example comprise a cyclotron, a synchrotron, a synchrocyclotron, a linear accelerator or any type of accelerator accelerating particles for providing an energetic particle beam.

Under particle beam is understood a charged hadron beam, for example a proton beam or a carbon ion beam. The energy of the beam can vary, depending on the type of particle and on the penetration depth to be reached in the target. For example, for proton beams, the energy can vary between 50 MeV and 300 MeV.

The particle beam system and method according to the invention can be used for particle therapy in order to determine the penetration depth of a particle or a bunch of particles in a patient. The particle beam source has a time structure for delivering the particles or bunches of particles. For example when the accelerator is a cyclotron, the particle beam source has a time structure defined by the frequency of the RF accelerator structure. For example a cyclotron operating at an RF frequency of 100 MHz will provide bunches of particles every 10 ns.

The particle beam system further comprises a timing controller configured for providing a timing reference signal representing the time structure for delivering the energetic particles or bunches of energetic particles. The timing reference can be a signal directly obtained from the RF structure of the accelerator which is representing the repetition of the delivery of the particle bunches.

In another embodiment, an additional beam buncher can be used to adapt time structure for delivering the bunches of particles. The beam buncher can for example optimize the length of the bunch. Preferably, the width of the bunch of the particles is smaller than 200 ps (picoseconds) and more preferably the width of the bunch is equal or smaller than 100 ps (picoseconds).

Alternatively, a detector can be used to intercept the particles of the beam and to generate a timing start signal each time a particle is intercepted. Such a detector is sometimes called a hodoscope. By intercepting particle per particle and associating a start signal to each particle, a timing reference signal representing the time structure for delivering the particles is obtained on a particle per particle base.

The particle beam system according to the invention further comprises beam direction means 80 for directing the particles or bunches of particles in a beam direction 60 pointing the target 20. Such a directing means can for example comprise a beam line to transport the beam and a scanning magnet for delivering the particles or bunches of particles to the target. The delivery means can also comprise a gantry rotatable around the target so as to direct the beam from different rotational angles to the target.

The particle beam system further comprises a first gamma detector 40 configured for detecting prompt gammas from the target and a second gamma detector 50 configured for detecting prompt gammas from the target. The first detector and the second detector are located at a different location with respect to the beam direction 60. The first and second detector are located such that the first and the second detector are oriented for detecting prompt gammas emitted at different angles with respect to the beam direction, as illustrated in Fig. 6.

The particle beam system further comprises a data acquisition system 90 for measuring prompt gammas in synchrony with the timing reference signal so as to obtain a timing profile indicating a number of prompt gammas measured as a function of the time elapsed since the delivery of a particle or a bunch of particles. The first and second detector are coupled to the data acquisition system so as to acquire a first timing profile from the first detector and to acquire a second timing profile from the second detector.

The particle beam system according to the invention comprises a data analyzer 100 comprising an algorithm configured for determining from the first timing profile a first time width $\Delta T1$ and determining from the second timing profile a second time width $\Delta T2$. The algorithm is further configured for determining a photon travel shift $\Delta DP$ defined as the distance travelled by a photon in the time interval equal to the difference between the first and the second time width, i.e. $\Delta DP = (\Delta T2 - \Delta T1)*c$, whereby c is equal to the speed of light. The algorithm of the data analyzer 100 is further configured for determining a penetration depth in the target by correlating the photon travel shift $\Delta DP$ with the difference in detector location between the first and second detector.

Under correlating the photon travel shift $\Delta DP$ with the difference in detector location between the first and second detector is understood that the photon travel shift can be expressed as depending on one hand on the distances the prompt gammas have to travel from the target to the first and second detector and on the other hand depending on a distance R in the target corresponding to maximum depth in the target from where prompt gammas have been emitted. It is well known in the art that this distance R is closely related to the penetration depth of the particle beam in the target as shown for example in Fig.2 (a) of Gueth et al, Phys. Med. Biol. 58 (2013) pages 4563 to 4577.

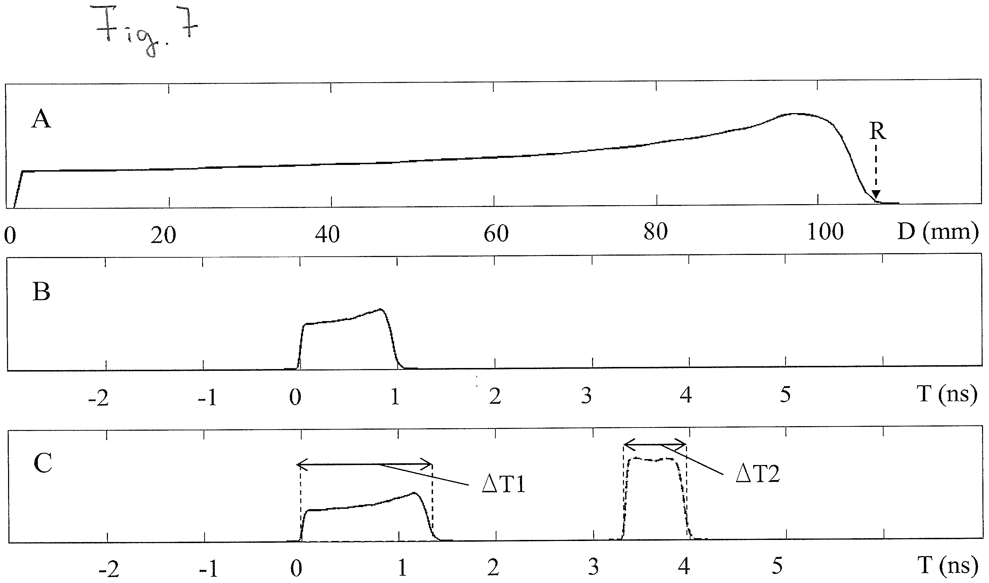

The invention is further illustrated in Fig. 7 which corresponds to a simulation of a 120 MeV proton beam in a tissue target. In this example, the first detector was located at angle of 180° with respect to the beam direction and the second detector was located at an angle of 0° with respect to the beam direction. The first detector was put on the front side of the target (distance zero) and the second detector was put at a distance of 100 cm from the target entrance. The top panel A shows the relative number of prompt gamma emissions as a function of the depth D in the target, the depth is expressed in mm. The depth R where no further prompt gammas are emitted is indicated on the figure. The middle panel B shows the relative number of prompt gammas emitted as function of time after entering the target. In this example, as can be seen on the middle panel B, prompt gammas are emitted during a time period of about 1 ns (one nanosecond). This time period correspond to the time the particle beam travels in the target until it is stopped when it has lost all its energy in the target.

The lower panel C of Fig. 7 shows the timing profiles as detected with the first detector and the second detector by measuring the prompt gammas in synchrony with the timing reference signal representing the time structure of the particle beam delivered to the target. As can be seen, the widths of the two timing profiles $\Delta T2$ and $\Delta T1$ are different. In this example, the two timing profiles in the lower panel C are clearly separated in time due to the large distance of the second detector from the target. In other examples, the second detector can also be put much closer to the target in which case the two timing profiles may overlap.

The correlation between the photon travel shift $\Delta DP$ and the difference in detector location between the first and second detector can be understood from the illustrative geometry shown in Fig. 6. The first time width $\Delta T1$, measured by the first detector is the difference in time a prompt gamma photon has to travel when either emitted at the entrance of target or emitted at the maximum depth R in the target where prompt gammas are emitted. Following the geometry comprising the distances d1, d2, d'1, d'2 and the two angles $\alpha$ and $\beta$ illustrated in Fig. 6, the time widths can be expressed as follows:

$$\Delta T1 = d1/c - (Tbeam + d'1/c) \qquad (Eq. 1)$$

$$\Delta T2 = d2/c - (Tbeam + d'2/c) \qquad\qquad (Eq.\ 2)$$

Whereby Tbeam is the time the particle beam travels in the target until it stops when having lost all its energy. This is the penetration depth of the particle beam in the target. As discussed before, the penetration depth of the particle beam is very closely related to the maximum depth position R of emission of prompt gammas. In the equations (1) and (2), c is the speed of light, d1 is the distance from the first detector to the entrance point, and d2 is the distance from the second detector to the entrance point. The entrance point is defined as the point where the particle beam enters the target. As further illustrated in Fig. 6, d'1 is the distance between the maximum depth R where prompt gammas are emitted and the first detector while d'2 is the distance between the maximum depth R and the second detector. By taking the difference between $\Delta T2$ and $\Delta T1$, one obtains that

$$\Delta T2 - \Delta T1 = (d2/c) - Tbeam - (d'2/c) - (d1/c) + Tbeam + (d'1/c)$$

or

$$\Delta T2 - \Delta T1 = (d2/c) - (d'2/c) - (d1/c) + (d'1/c) \qquad\qquad (Eq.\ 3)$$

In other words, this time difference is independent from the particle beam travel time Tbeam in the target.

As discussed above, a photon travel shift $\Delta DP$ can be defined as

$$\Delta DP = (\Delta T2 - \Delta T1)^*c$$

or

$$\Delta DP = d2 - d'2 - d1 + d'1 \qquad\qquad (Eq.\ 4)$$

Following standard trigonometric rules one finds that

$$\Delta DP = \quad SQRT(R*R + d1*d1 - 2*R*d1*\cos(\alpha)) -$$
$$SQRT(R*R + d2*d2 - 2*R*d2*\cos(\beta)) - (d1-d2) \qquad (Eq.\ 5)$$

Whereby, as discussed above, R is a distance in the target corresponding to maximum depth in the target from where prompt gammas have been emitted. This is the unknown distance in the target that can be determined with the device and method of the invention. To find R from the above equation number (5), the inventors have used a numerical method to find R.

Preferably, the data analyzer 100 according to the invention comprises computing means configured for calculating this distance R in the target by solving the equation number (5) deduced above.

The width $\Delta T1$ and the width $\Delta T2$ can be determined in various ways. For example, the timing profiles can be fitted with a curve and the begin and end point of the profile can be deduced. Alternatively, the width can be defined by defining the begin point and the end point of the profile as being a point having a given value with respect to the average peak value, for example defining the begin and end point as being the 5% level points.

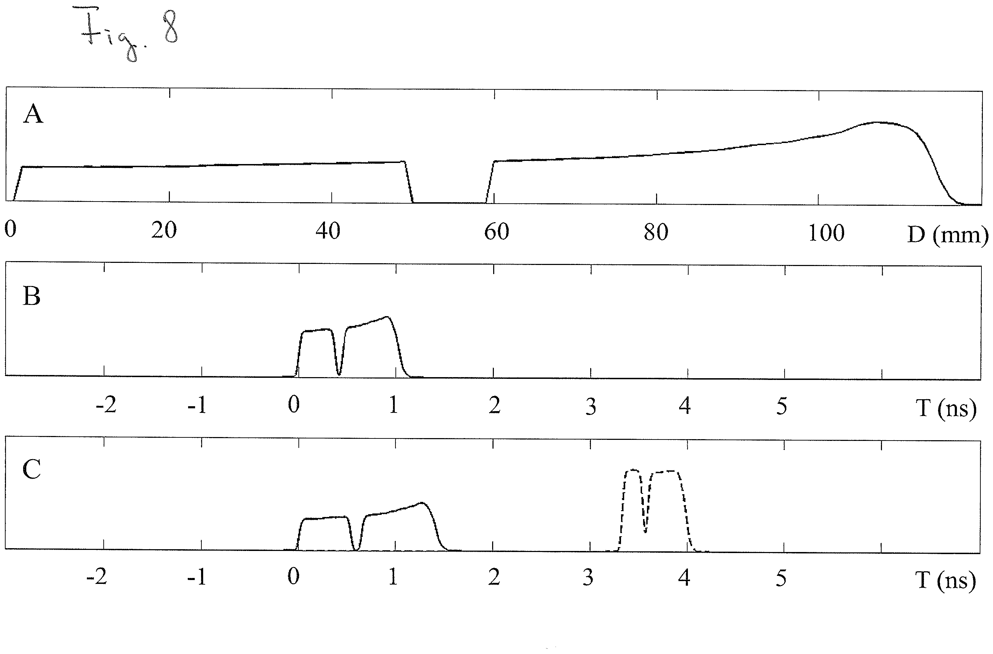

The inventors have investigated the robustness of the particle beam system according to the invention with respect to inhomogeneities in the target. As an example, the effect of an inhomogeneity is shown in Fig. 8 where an air gap with a thickness 10 mm is located at a depth in the target of 50 mm. In the top panel A, the number of prompt gammas produced as function of the penetration depth in the target are shown. At the location of the air gap, as shown, no prompt gammas are produced. This is then also observed in the middle panel B where the relative number of prompt gammas emitted as function of time after entering the target is plotted. The emission profile in the second panel B is showing a longer emission profile compared with the emission profile of Fig. 7. The third panel C shows that the air gap has also an effect on the shape of the first timing profile and the second timing profile.

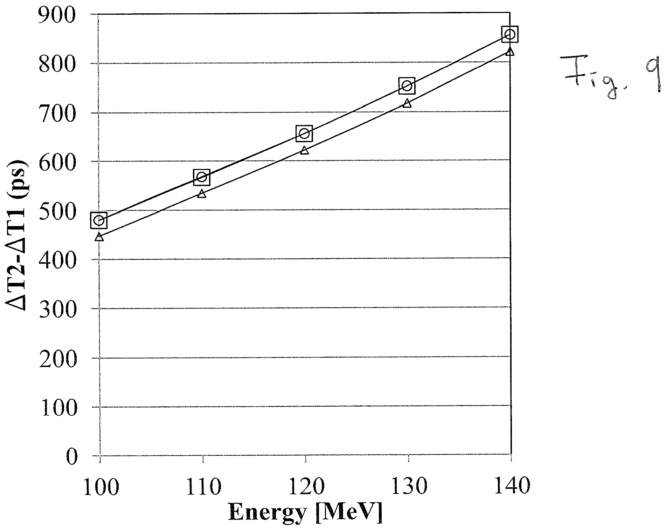

It has been found by the inventors that the location of inhomogeneities in the target have no effect on the accuracy for determining the particle beam depth. This is has been examined and is illustrated in Fig. 9 where $\Delta T2 - \Delta T1$ is plotted for proton beams in a plastic target (more specifically a PMMA) having energies between 100 MeV and 140 MeV. Three cases have been evaluated and the results are plotted on the same Fig. 9: 1) circles correspond to a situation where there is a 5 mm cavity located at 1 cm depth in the target, 2) squares correspond to a situation where there is a 5 mm cavity located at 6 cm depth in the target and 3) triangles correspond to a situation where there is no cavity in the target. These results show that not only the 5 mm shift due to the cavity is clearly observed but also that there is no difference for what concerns the location of the cavity so that the particle depth determining method according to the invention shows to be robust.

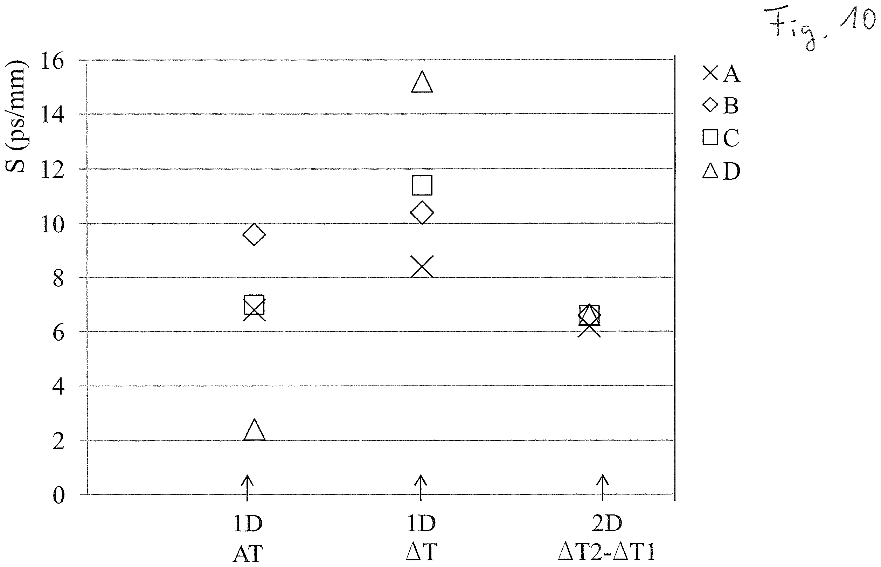

The robustness of the particle beam system and the method according to the invention has been further investigated by performing additional simulations. For the exemplary simulation, a beam of 140 MeV of protons was sent on a plastic target (PMMA). The beam was a bunched beam with a bunch duration of 40 ps (picoseconds). The expected particle penetration depth for a homogeneous target having an average density of 1,19 g/cm3 (=reference target) is 12,1 cm. To test the robustness of the method of range verification, four different modifications were made to the target to induce a same 5 mm (millimeter) shift of the penetration depth of the particle beam. The purpose of the test is to demonstrate that the system and method according to the invention can measure such a shift in penetration depth independent of the nature that causes the range shift. Those four modifications made to the target to induce a 5 mm shift in penetration depth, as indicated in Fig. 10, are the following: A) indicated with a cross, is related to target having an overall reduction of the density by 0,05 g/cm3 compared to the reference target, i.e. an average target density of 1,14 g/cm3 instead of the 1,19 g/cm3 for the reference target, B) indicated with a diamond, is related to a target having a cavity of 5mm located at a depth of 1 cm in the target, C) indicated with a square, is related to a target having a cavity of 5mm located at a depth of 6 cm in the target, D) indicated with a triangle, is related to a target having a cavity of 5mm located at a depth of 11 cm in the target. For the four cases A, B, C, D, it is expected that the proton penetration depth shifts with 5 mm compared to the reference target. The purpose of this test was to verify the sensitivity to detect such variations in penetration depth. The sensitivity is defined as the variation of a measured time signal per mm of change of penetration.

The results of the exemplary test simulations are shown in Fig. 10. For this exemplary simulation, a first detector was located at 180° and a second detector at 0° with respect to the beam direction. According to the invention, the values $\Delta T2 - \Delta T1$ were determined for timing profiles obtained with the reference target and with the four modified targets (the cases A, B, C or D as discussed above). The sensitivity S of the detection method according to the invention is obtained by first calculating ($\Delta T2 - \Delta T1$) measured for the four modified targets (A, B, C or D) and subtracting the ($\Delta T2 - \Delta T1$) determined for the reference target and then in a second step dividing this difference in time by the expected penetration shift of 5 mm. This sensitivity S, expressed in ps (picoseconds) per mm (millimeter) is plotted in Fig. 10 on the right side, at the coordinate position labeled "2D $\Delta T2 - \Delta T1$". As shown in this example, the sensitivity using the method according to the invention is about 6,5 ps/mm. This means that one has to measure a time difference of 6,5 ps to detect a variation in penetration depth of one mm. What is important is that the same sensitivity is obtained for the four cases A, B, C, D, i.e. the sensitivity is independent of the nature of what caused the shift in penetration depth (e.g. an overall density change, a cavity in the target, ...).

To stress this important advantage of the invention where a particle penetration depth is verified independently of the nature causing a shift in penetration depth, an additional simulation is performed for a system and method that would only use one detector to measure the prompt gammas and hence only obtaining one prompt gamma timing profile. The results are also shown in Fig. 10 where at the coordinate position labeled "1D $\Delta T$", the results of the sensitivity of a single detector are shown. In this case, the sensitivity is obtained by, in a first step, determining the difference between the width $\Delta T$ measured for a modified target (the case A, B, C or D discussed above) and subtracting the width $\Delta T$ determined for the reference target and then, in a second step, dividing this difference in time by the expected penetration shift of 5 mm. The width $\Delta T$ is defined and determined in the same way as was done for $\Delta T1$ and $\Delta T2$, discussed above (equation (1) or (2)). As shown in Fig. 10, for each of the four modified target cases A, B, C and D, four different sensitivity values S are obtained. In other words, when using only one detector for detecting the prompt gammas, the determination of penetration depth requires an exact knowledge of the target structure. For example, if there are cavities in the target one needs to know where they are located in the target.

The reason of this sensitivity variation observed when using only one detector and associated timing profile can be understood from equation (1) or (2), where it is shown that $\Delta T$ depends on Tbeam, the time the particle has to travel in the target. This particle travel time depends on the target structure. As the particle speed decreases when penetrating the target, the travel time will be different when for example a cavity is present in the distal part or the proximal part of the target. With the system and method according to the invention the penetration depth is deduced from a time difference $\Delta T2 - \Delta T1$ independent from Tbeam.

Alternatively, when using only one detector, instead of determining the width $\Delta T$, one could also determine an average value "AT" of the timing profile. Similarly, the average value AT obtained for the reference target can then be compared with the value obtained for the modified targets (A, B, C, D as discussed above). The results of this approach are also shown in Fig. 10 at the coordinate position labeled "1D AT". As shown, also with this approach the sensitivity for detecting a penetration depth variation is dependent on the target structure.

The timing setup and technology for measuring the two timing profiles with the first and the second detector can adopt technology that is currently used in standard time-of-flight (TOF) measurements used in for example particle beam physics or used for PET time-of-flight measurements (see e.g. Schaart et al. in Phys. Med. Biol. 55 (2010) N179-N189). The first gamma detector and the second gamma detector can be gamma detectors well known in the art such as for example detectors comprising a scintillation crystal and a photomultiplier readout.

With the particle beam system according to the invention, the timing profiles are preferably measured with a timing resolution equal or less than 10 ns. In this way, when accounting for sufficient statistics in the timing profiles (for example, a few 100 to 1000 counts), time differences in the ps (picoseconds) time range can be measured. More preferably, the timing profiles are measured with a timing resolution equal or smaller than 1 ns.

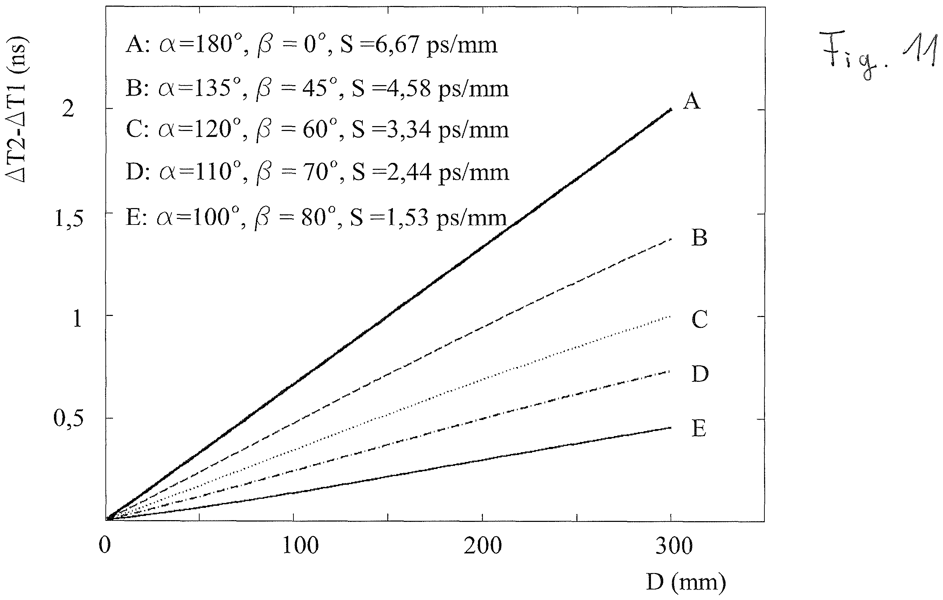

The relative geometry of the first and second detector is further discussed and illustrated in Fig. 11. According to the invention, the first and second detector are positioned at a different angle with respect to the beam direction. The method of the invention can be applied for any position of the two detectors, as long as the two detectors measure prompt gammas emitted from a different direction with respect to the beam direction. In Fig. 11, the time difference $\Delta T2 - \Delta T1$ is plotted as function of the penetration depth D of the beam in the target for different detector setups labelled A, B, C, D, E. Each detector setup is defined by the angles $\alpha$ and $\beta$ defining the angle between the beam direction and the direction from the target to respectively the first and the second detector. The average sensitivity S for each of the configurations is also plotted on Fig. 11. The highest sensitivity of 6,67 ps/mm is obtained for case A where the two detectors are separated by 180° (degree) and the lowest sensitivity of 1,53 ps/mm is obtained for case E where the two detectors are separated by an angle of 20°.

Preferably, the particle beam system according to the invention has the second gamma detector 50 located with respect to the first gamma detector 40 such that the first and the second detector are detecting prompt gammas emitted at angles that differ by at least 20°. The angles of emission of the prompt gammas are defined with respect to the beam direction.

More preferably, the first gamma detector is configured for detecting prompt gammas emitted at an angle equal or larger than 100° with respect to the beam direction and the second gamma detector is configured for detecting prompt gammas emitted at an angle equal or smaller than 80° with respect to the beam direction.

As already indicated above, according to a second aspect of the invention, a method is provided to determine a penetration depth of an energetic particle beam in a target by detecting prompt gammas produced when the energetic particle beam penetrates the target. As also discussed above, a particle beam is formed by particles or a bunch of particles.

As schematically illustrated in Fig. 6, a first gamma detector 40 and a second gamma detector 50 are provided for detecting prompt gammas emitted from the target. In the method according to the invention, the two prompt gamma detectors are located in a different location with respect to the target such that the first and the second detector are configured for detecting prompt gammas emitted at different angles with respect to the beam direction.

Further, a timing reference signal representing a time structure of the energetic particle beam is provided. According to the method of the invention, prompt gammas are measured with the first detector in synchrony with the timing reference signal so as to obtain a first timing profile. Similar, with the second detector, prompt gammas are measured with the second detector in synchrony with the timing reference signal so as to obtain a second timing profile.

According to the method of the invention, a first time width $\Delta T1$ is determined from the first timing profile and a second time width $\Delta T2$ is determined from the second timing profile. In a next step, a photon travel shift $\Delta DP$ is determined whereby the photon travel shift is defined as the distance travelled by a photon in the time interval equal to the difference between the first and the second time width: $\Delta DP = (\Delta T2 - \Delta T1)*c$ whereby c is equal to the speed of light.

Finally, according to the method of the invention, the penetration depth is determined by correlating the photon travel shift $\Delta DP$ with the difference in detector location between the first and second detector.

Preferably, the method of the invention comprises a calculating step for calculating a distance R in the target by solving the equation

$$\Delta DP = \text{SQRT}(R*R + d1*d1 - 2*R*d1*\cos(\alpha)) -$$
$$\text{SQRT}(R*R + d2*d2 - 2*R*d2*\cos(\beta)) - (d1\text{-}d2)$$

Whereby $\Delta DP$ is the said photon travel shift, d1 is the distance from the first detector to the entrance point, the entrance point being defined as the point where the energetic particle beam enters the target, d2 is the distance from the second detector to the entrance point, $\alpha$ is the angle between the beam direction and the direction going from the entrance point towards the first detector, and $\beta$ is the angle between the beam direction and the direction going from the entrance point towards the second detector.

Preferably, in the method according to the invention, the second gamma detector 50 is located with respect to the first detector 40 such that the first and the second detector are detecting prompt gammas emitted at angles with respect to the beam direction 60 that differ by at least 20°.

More preferably, in the method according to the invention, the first gamma detector 40 is located for measuring prompt gammas emitted at an angle equal or larger than 100° with respect to the beam direction and the second gamma detector 50 is located for measuring prompt gammas emitted at an angle equal or smaller than 80° with respect to the beam direction.

**Claims**

1. Method for the verification of the range of a charged hadronic particle radiation (3) of a radiation device (1) for radiation therapy by measuring prompt gamma radiation being produced by the charged hadronic particle radiation (3) when passing tissue of an object to be radiated with at least one detector (2) being able to detect single gamma particles (4) of the prompt gamma radiation and at least one analyzer (7), comprising the following steps:

   - when detecting a gamma particle (4), furthermore denominated as event, a signal is created in the detector (2), whereby the signal is correlated in time with the arrival of the gamma particle (4) in detector (2),
   - the analyzer (7), analyzing the signal of the detector (2), does assign either every event or selected events a time of detection,
   - the radiation device (1) or a separate particle detector (8) does provide a reference signal, which is correlated to the emersion of single particles or particle bunches from the radiation device (1) out of the radiation device (1) with an uncertainty in time of equal or smaller than 10ns,
   - a time t1 is obtained from the reference signal for each event or each of the selected events in the analyzer (7), at which the particle or the particle bunch, which has produced the gamma particle (4) detected by the detector (2), further described as event triggering particle or particle bunch, has passed a reference plane perpendicular to the direction of the beam of the radiation device (1),
   - a time t2 is obtained from the detector (2), corresponding to the striking of the gamma particle (4) in detector (2)
   - the time difference t between the time of the detection of the gamma particle (4) in the detector (2) t2 and the time of the passing of the reference plane by the event triggering particle or particle bunch t1 is calculated for each event or the selected events within the analyzer (7), said time difference being a measure for the length of the flight path of the charged hadronic particle radiation (3),
   - at least one statistic distribution (6) of the time differences t is obtained in the analyzer (7), furthermore described as time distribution (6),

   **characterized in that** the method further comprises the following steps:

   - integral parameters are determined from the time distribution (6), wherein the integral parameters represent information about the range of the charged hadronic particle radiation (3) in the object to be radiated , wherein the integral parameters are compared with a time distribution of a reference to determine movements in the range of the charged hadronic particle radiation in the object to be radiated, so that a statement with respect to the conformity of the radiation therapy to be verified with the apparatus with the therapy plan can be obtained mainly from that time distribution (6), and that
   - the analyzer (7) does analyze the time distribution (6), thereby deriving information about the range of the particle beam (3), so that a statement regarding the conformity with the therapy to be verified by the device with the radiation plan is given in real time, said statement being derived from an integral parameter of the time distribution (6).

2. Method according to claim 1, **characterized in that** the analyzer (7) does select events, from which the time distribution (6) is created according to predetermined criteria to the signal of the detector (2), those predetermined criteria preferably being selected from a group of the

   - magnitude,
   - amplitude, and/or
   - shape of the timing signal of the detector (2).

3. Method according to at least one of claims 1 to 2, **characterized in that** the radiation device does comprise at least a second gamma detector for detecting prompt gammas emitted from said target, the second detector located with respect to the first detector such that the first and the second detector are detecting prompt gammas emitted from different angles with respect to said beam direction, obtaining a first time distribution (6) for the first gamma detector and obtaining a second time distribution (6) for the second gamma detector, determining from said first time distribution a first time width $\Delta T1$, determining from said second time distribution a second time width $\Delta T2$, determining a photon travel shift $\Delta DP$, defined as a distance travelled by a photon in the time interval equal to the difference between the first and the second time width multiplied by the speed of light c: $\Delta DP = (\Delta T2 - \Delta T1)^*c$, and determining the range of the particle beam by correlating said photon travel shift $\Delta DP$ with the difference in detector location between the first and the second detector.

4. A method according to claim 3 whereby the step of determining said penetration depth comprises a step of calculating a distance R in the target by solving the equation

$$\Delta DP = \quad SQRT(R*R + d1*d1 - 2*R*d1*cos(\alpha)) -$$

$$SQRT(R*R + d2*d2 - 2*R*d2*cos(\beta)) - (d1-d2),$$

whereby

$\Delta DP$ is the said photon travel shift;
d1 is the distance from the first detector to the entrance point, the entrance point being defined as the point where the energetic particle beam enters the target;
d2 is the distance from the second detector to the entrance point;
$\alpha$ is the angle between the beam direction and the direction going from the entrance point towards the first detector;
$\beta$ is the angle between the beam direction and the direction going from the entrance point towards the second detector.

5. A method according to any of claims 3 or 4 wherein said second gamma detector is located with respect to said first detector such that the first and the second detector are detecting prompt gammas emitted at angles with respect to the beam direction (6) that differ by at least 20°.

6. A method according to any of claims 3 or 4 wherein said first gamma detector is located for measuring prompt gammas emitted at an angle equal or larger than 100° with respect to the beam direction and said second gamma detector is located for measuring prompt gammas emitted at an angle equal or smaller than 80° with respect to the beam direction.

7. Apparatus for verifying the range of charged hadronic particle radiation (3) from a radiation device (1) for radiation therapy, measuring prompt gamma radiation being produced by the charged hadronic particle radiation when passing tissue of an object to be radiated, configured to perform a method according to one of claims 1 to 6 with at least one first detector (2), set up to detect single gamma particles of the prompt gamma radiation and at least one analyzer (7), the detector (2) being a signal generating detector (2) set up to generate a signal following the detection of a gamma particle (4), said detection furthermore described as event, said signal being correlated with the arrival of the gamma particle (4) at the detector (2), whereby the detector (2) is connected to the analyzer (7), whereby the analyzer (7) is configured to analyze the signal from the detector (2) and to assign either each event or selected events a time of its detection, whereby the analyzer (7) is further connected to the radiation device (1) or a separate particle detector (8), whereby the radiation device (1) or the particle detector (8) is configured to provide a reference signal, which is correlated with the emission of single particles or particle bunches from the radiation device (1) out of the radiation device (1), and that the analyzer (7) is an analyzer (7),

- configured to determine a time from the reference signal for either each event or selected events, at which the particle or the particle bunch, which has created the gamma particle (4) being detected in the detector (2), referenced as event setting particle or particle bunch, has passed a reference plane being perpendicular to the beam exit of the radiation device (1),
- is configured to calculate a time difference $\Delta t1$ between the time of detection of the gamma particle (4) in the detector (2) and the time of passing of the reference plane by the event setting particle or particle bunch for each event or the selected events, said time difference being a measure for the length of the flight path of the charged hadronic particle radiation (3),
- is configured to derive at least one statistic distribution (6) of time differences, referenced as time distribution (6), **characterized in that** the analyzer is further configured
- to determine integral parameters from the time distribution (6), wherein the integral parameters represent information about the range of the charged hadronic particle beam (3) and to compare the integral parameters with a time distribution of a reference and configured to determine movements in the range of the charged hadronic particle radiation in the object to be radiated,

thus allowing for a statement about the conformity of the therapy, being verified with the apparatus, with the radiation plan mainly based on this time distribution (6).

8. Apparatus according to claim 7, **characterized in that** the detector (2), being able to detect single gamma particles (4), is

   - a scintillation detector (2), comprising of at least one scintillator and at least one photo detector,
   - a Cherenkov detector (2), comprising of at least one photodetector, coupled to a transparent medium, or a
   - Resistive Plate Chamber (RPC) (2).

9. Apparatus according to claim 7, **characterized in that** said apparatus further comprises a second gamma detector, configured for detecting prompt gammas emitted from said target, being located with respect to said first detector such that the first and the second detector are detecting prompt gamma particles emitted from different angles with respect to the beam direction, the analyzer calculating the time differences $\Delta t1$ and $\Delta t2$ for both detectors so as to obtain time distributions for both detectors, further comprising an analyzer comprising an algorithm configured for determining from said first time difference $\Delta t1$ a first time width $\Delta T1$ and from said second time difference $\Delta t2$ a second time width $\Delta T2$, further determining a photon travel shift $\Delta DP$ defined as a distance travelled by a photon in the time interval equal to the difference between the first and the second time width: $\Delta DP = (\Delta T2 - \Delta T1)*c$, whereby c is equal to the speed of light, determining a particle beam penetration depth by correlating said photon travel shift $\Delta DP$ with the difference in detector location between the first and second detector.

10. An apparatus according to the previous claim, **characterized in that** the analyzer comprises computing means configured for calculating a distance R in the target by solving the equation

$$\Delta DP = SQRT(R*R + d1*d1 - 2*R*d1*\cos(\alpha)) -$$
$$SQRT(R*R + d2*d2 - 2*R*d2*\cos(\beta)) - (d1-d2),$$

whereby

   $\Delta DP$ is the said photon travel shift;
   d1 is the distance from the first detector to the entrance point, the entrance point being defined as the point where the energetic particle beam enters the target;
   d2 is the distance from the second detector to the entrance point;
   $\alpha$ is the angle between the beam direction and the direction going from the entrance point towards the first detector;
   $\beta$ is the angle between the beam direction and the direction going from the entrance point towards the second detector.

11. An apparatus according to any of previous claims 10 to 11 wherein said second gamma detector is located with respect to said first gamma detector such that the first and the second gamma detector are detecting prompt gammas emitted at angles with respect to the beam direction that differ by at least 20°.

12. An apparatus according to any of previous claims 9 to 11 wherein said first gamma detector is located for measuring prompt gammas emitted at an angle equal or larger than 100° with respect to the beam direction and said second gamma detector is located for measuring prompt gammas emitted at an angle equal or smaller than 80° with respect to the beam direction.

**Patentansprüche**

1. Verfahren für die Verifizierung der Reichweite einer geladenen hadronischen Teilchenstrahlung (3) einer Strahlungsvorrichtung (1) für die Strahlentherapie durch Messen von prompter Gammastrahlung, die durch die geladene hadronische Teilchenstrahlung (3) beim Passieren von zu bestrahlendem Gewebe erzeugt wird, mit mindestens einem Detektor (2), der in der Lage ist, einzelne Gamma-Teilchen (4) der prompten Gammastrahlung zu erfassen und mindestens einem Analysator (7), umfassend die folgenden Schritte:

   - beim Erfassen eines Gammateilchens (4), im Weiteren als Ereignis bezeichnet wird, ein Signal in dem Detektor (2) erstellt, wobei das Signal mit der Ankunft des Gammateilchens (4) in dem Detektor (2) zeitlich korreliert ist,
   - der Analysator (7), der das Signal des Detektors (2) analysiert, ordnet entweder jedem Ereignis oder ausgewählten Ereignissen einen Erfassungszeitpunkt zu,

- die Strahlungsvorrichtung (1) oder ein separater Teilchendetektor (8) stellt ein Referenzsignal bereit, das mit der Emersion einzelner Teilchen oder Teilchenbündel von der Strahlungsvorrichtung (1) aus der Strahlungsvorrichtung (1) mit einer zeitlichen Unschärfe von gleich oder kleiner als 10 ns korreliert ist,

- ein Zeitpunkt t1 wird von dem Referenzsignal für jedes Ereignis oder jedes der ausgewählten Ereignisse in dem Analysator (7) erhalten, zu dem das Teilchen oder das Teilchenbündel, das das Gammateilchen (4), das durch den Detektor (2) erfasst wird, erzeugt hat, im Weiteren beschrieben als Ereignis auslösendes Teilchen oder Teilchenbündel, eine Referenzebene, die senkrecht zu der Richtung des Strahls der Strahlungsvorrichtung (1) ist, passiert hat,

- ein Zeitpunkt t2 wird von dem Detektor (2) erhalten, der dem Auftreffen des Gammateilchens (4) in dem Detektor (2) entspricht.

- der Zeitunterschied t zwischen dem Zeitpunkt der Erfassung des Gammateilchens (4) in dem Detektor (2) t2 und dem Zeitpunkt des Passierens der Referenzebene durch das Ereignis auslösende Teilchen oder Teilchenbündel t1 auslöst wird, für jedes Ereignis oder ausgewählte Ereignisse innerhalb des Analysators (7) berechnet, wobei der Zeitunterschied ein Maß für die Länge der Flugbahn der geladenen hadronischen Teilchenstrahlung (3) ist,

- mindestens eine statistische Verteilung (6) der Zeitunterschiede wird in dem Analysator (7) erhalten, im Weiteren als Zeitverteilung (6) beschrieben,

**dadurch gekennzeichnet, dass** das Verfahren ferner die folgenden Schritte umfasst:

- Integralparameter werden aus der Zeitverteilung (6) bestimmt, wobei die Integralparameter Informationen über die Reichweite der geladenen hadronischen Teilchenstrahlung (3) in dem zu bestrahlenden Objekt repräsentieren, wobei die Integralparameter mit einer Zeitverteilung einer Referenz verglichen werden, um Veränderungen in der Reichweite der geladenen hadronischen Teilchenstrahlung in dem zu bestrahlenden Objekt zu bestimmen, so dass eine Aussage in Bezug auf die Konformität der mit der Vorrichtung zu verifizierenden Strahlentherapiemit dem Therapieplan im Wesentlichen aus dieser Zeitverteilung erhalten werden kann, und dass

- der Analysator (7) die Zeitverteilung (6) analysiert, wodurch Informationen über die Reichweite des Teilchenstrahls (3) abgeleitet werden, so dass eine Aussage bezüglich der mit der Vorrichtung zu verifizierenden Konformität der Therapie mit dem Bestrahlungsplan in Echtzeit gegeben wird, wobei die Aussage von einem integralen Parameter der Zeitverteilung (6) abgeleitet wird.

2. Verfahren nach Anspruch 1, **dadurch gekennzeichnet, dass** der Analysator (7) Ereignisse auswählt, aus denen die Zeitverteilung (6) anhand vorherbestimmter Kriterien zu dem Signal des Detektors (2) erstellt wird, wobei diese vorherbestimmten Kriterien vorzugsweise ausgewählt werden aus einer Gruppe der

- Größe,
- Amplitude und/oder
- Form des Zeitsignals des Detektors (2).

3. Verfahren nach mindestens einem der Ansprüche 1 bis 2, **dadurch gekennzeichnet, dass** die Strahlungsvorrichtung mindestens einen zweiten Gammadetektor zum Erfassen von prompten Gammas, die von dem Ziel emittiert werden, umfasst, wobei der zweite Detektor in Bezug auf den ersten Detektor derart lokalisiert ist, dass der erste und der zweite Detektor prompte Gammas erfassen, die aus unterschiedlichen Winkeln in Bezug auf die Strahlrichtung emittiert werden, wobei eine erste Zeitverteilung (6) für den ersten Gammadetektor erhalten wird und eine zweite Zeitverteilung (6) für den zweiten Gammadetektor erhalten wird, wobei aus der ersten Zeitverteilung eine erste Zeitbreite $\Delta T1$ bestimmt wird, wobei aus der zweiten Zeitverteilung eine zweite Zeitbreite $\Delta T2$ bestimmt wird, wobei eine Photonenlaufverschiebung $\Delta DP$ bestimmt wird, die als eine von einem Photon in dem Zeitintervall zurückgelegte Distanz definiert ist, die gleich dem Unterschied zwischen der ersten und der zweiten Zeitbreite multipliziert mit der Lichtgeschwindigkeit c ist: $\Delta DP = (\Delta T2 - \Delta T1)*c$, und Bestimmen der Reichweite des Teilchenstrahls durch Korrelieren der Photonenlaufverschiebung $\Delta DP$ mit dem Unterschied in dem Detektorstandort zwischen dem ersten und dem zweiten Detektor.

4. Verfahren nach Anspruch 3, wobei der Schritt des Bestimmens der Eindringtiefe einen Schritt des Berechnens einer Distanz R in dem Ziel durch Lösen der folgenden Gleichung umfasst:

$$\Delta DP = SQRT(R*R + d1*d1 - 2*R*d1*\cos(\alpha)) - SQRT(R*R + d2*d2 - 2*R*d2*\cos(\beta)) - (d1 - d2),$$

wobei

$\Delta$DP die Photonenlaufverschiebung ist;

d1 die Distanz von dem ersten Detektor zu dem Eintrittspunkt ist, wobei der Eintrittspunkt als der Punkt definiert ist, an dem der Strahl energiereicher Teilchen in das Ziel eintritt;

d2 der Abstand von dem zweiten Detektor zu dem Eintrittspunkt ist;

$\alpha$ der Winkel zwischen der Strahlrichtung und der Richtung, die von dem Eintrittspunkt zu dem ersten Detektor hin verläuft, ist;

$\beta$ der Winkel zwischen der Strahlrichtung und der Richtung, die von dem Eintrittspunkt zu dem zweiten Detektor hin verläuft, ist.

5. Verfahren nach einem der Ansprüche 3 oder 4, wobei der zweite Gammadetektor in Bezug auf den ersten Detektor derart lokalisiert ist, dass der erste und der zweite Detektor prompte Gammas erfassen, die in Winkeln in Bezug auf die Strahlrichtung (6) emittiert werden, die sich um mindestens 20° unterscheiden.

6. Verfahren nach einem der Ansprüche 3 oder 4, wobei der erste Gammadetektor zum Messen von prompten Gammas lokalisiert ist, die in einem Winkel von gleich oder größer als 100° in Bezug auf die Strahlrichtung emittiert werden, und wobei der zweite Gammadetektor zum Messen von prompten Gammas lokalisiert ist, die in einem Winkel von gleich oder kleiner als 80° in Bezug auf die Strahlrichtung emittiert werden.

7. Vorrichtung zum Verifizieren der Reichweite von geladener hadronischer Teilchenstrahlung (3) aus einer Strahlungsvorrichtung (1) für die Strahlentherapie, wobei prompte Gammastrahlung gemessen wird, die durch die geladene hadronische Teilchenstrahlung beim Passieren von zu bestrahlendem Gewebe erzeugt wird, die konfiguriert ist, um ein Verfahren nach einem der Ansprüche 1 bis 6 durchzuführen, mit mindestens einem ersten Detektor (2), der eingerichtet ist, um einzelne Gammateilchen der prompten Gammastrahlung zu erfassen, und mindestens einem Analysator (7), wobei der Detektor (2) ein signalgenerierender Detektor (2) ist, der eingerichtet ist, um ein Signal, das der Erfassung eines Gammateilchens (4) folgt, zu generieren, wobei die Erfassung im Weiteren als Ereignis beschrieben wird, wobei das Signal mit der Ankunft des Gammateilchens (4) an dem Detektor (2) korreliert ist, wobei der Detektor (2) mit dem Analysator (7) verbunden wird, wobei der Analysator (7) konfiguriert ist, um das Signal von dem Detektor (2) zu analysieren und entweder jedem Ereignis oder ausgewählten Ereignissen einen Erfassungszeitpunkt zuzuordnen, wobei der Analysator (7) ferner mit der Strahlungsvorrichtung (1) oder einem separaten Teilchendetektor (8) verbunden ist, wobei die Strahlungsvorrichtung (1) oder der Teilchendetektor (8) konfiguriert sind, um ein Referenzsignal bereitzustellen, das mit der Emission einzelner Teilchen oder Teilchenbündel von der Strahlungsvorrichtung (1) aus der Strahlungsvorrichtung (1) korreliert ist, und dass der Analysator (7) ein Analysator (7) ist,

- der konfiguriert ist, um einen Zeitpunkt aus dem Referenzsignal für jedes Ereignis oder für ausgewählte Ereignisse zu bestimmen, zu dem das Teilchen oder das Teilchenbündel, das das Gammateilchen (4) erzeugt hat, in dem Detektor erfasst wird (2), referenziert als ereignissetzendes Teilchen oder Teilchenbündel eine Referenzebene passiert hat, die senkrecht zu dem Strahlenaustritt der Strahlungsvorrichtung (1) ist,

- der konfiguriert ist, um einen Zeitunterschied $\Delta$t1 zwischen dem Erfassungszeitpunkt des Gammateilchens (4) in dem Detektor (2) und dem Zeitpunkt des Passierens der Referenzebene durch das ereignissetzende Teilchen oder Teilchenbündel für jedes Ereignis oder die ausgewählten Ereignisse zu berechnen, wobei der Zeitunterschied ein Maß für die Länge der Flugbahn der geladenen hadronischen Teilchenstrahlung (3) ist,

- der konfiguriert ist, um mindestens eine statistische Verteilung (6) von Zeitunterschieden abzuleiten, referenziert als Zeitverteilung (6),

**dadurch gekennzeichnet, dass** der Analysator ferner konfiguriert ist zum

- Bestimmen von Integralparametern aus der Zeitverteilung (6), wobei die Integralparameter Informationen über die Reichweite des geladenen hadronischen Teilchenstrahls (3) darstellen, und um die Integralparameter mit einer Zeitverteilung einer Referenz zu vergleichen, und konfiguriert, um Veränderungen in der Reichweite der geladenen hadronischen Teilchenstrahlung in dem zu bestrahlenden Objekt zu bestimmen,

somit Ermöglichen einer Aussage über die Konformität der mit der Vorrichtung zu verifizierenden Therapie mit dem Bestrahlungsplan hauptsächlich basierend auf dieser Zeitverteilung (6),

8. Vorrichtung nach Anspruch 7, **dadurch gekennzeichnet, dass** der Detektor (2), der in der Lage ist, einzelne Gammateilchen (4) zu erfassen,

- ein Szintillationsdetektor (2), umfassend mindestens einen Szintillator und mindestens einen Photodetektor,
- ein Tscherenkow-Detektor (2), umfassend mindestens einen Photodetektor, der an ein transparentes Medium gekoppelt ist, oder eine
- Widerstandsplattenkammer (RPC) (2)

ist.

9. Vorrichtung nach Anspruch 7, **dadurch gekennzeichnet, dass** die Vorrichtung ferner einen zweiten Gammadetektor umfasst, der zum Erfassen von prompten Gammateilchen konfiguriert ist, die von dem Ziel emittiert werden, der in Bezug auf den ersten Detektor derart lokalisiert ist, dass der erste und der zweite Detektor prompte Gammateilchen erfassen, die aus unterschiedlichen Winkeln in Bezug auf die Strahlrichtung emittiert werden, wobei der Analysator die Zeitunterschiede Δt1 und Δt2 für beide Detektoren berechnet, um Zeitverteilungen für beide Detektoren zu erhalten, ferner umfassend einen Analysator, umfassend einen Algorithmus, der zum Bestimmen einer ersten Zeitbreite ΔT1 aus dem ersten Zeitunterschied Δt1, und einer zweiten Zeitbreite ΔT2 aus dem zweiten Zeitunterschied Δt2,, konfiguriert ist, wobei ferner eine Photonenlaufverschiebung ΔDP bestimmt wird, die als eine von einem Photon in dem Zeitintervall zurückgelegte Distanz definiert ist, die gleich dem Unterschied zwischen der ersten und der zweiten Zeitbreite ist: ΔDP = (ΔT2-ΔT1)*c, wobei c gleich der Lichtgeschwindigkeit ist, Bestimmen der Eindringtiefe eines Teilchenstrahls durch Korrelieren der Photonenlaufverschiebung ΔDP mit dem Unterschied in dem Detektorstandort zwischen dem ersten und dem zweiten Detektor.

10. Vorrichtung nach dem vorstehenden Anspruch, **dadurch gekennzeichnet, dass** der Analysator Rechenmittel umfasst, die zum Berechnen einer Distanz R in dem Ziel durch Lösen der folgenden Gleichung konfiguriert sind:

$$\Delta DP = SQRT(R*R + d1*d1 - 2*R*d1*\cos(\alpha)) -$$

$$SQRT(R*R + d2*d2 - 2*R*d2*\cos(\beta)) - (d1-d2),$$

wobei

ΔDP die Photonenlaufverschiebung ist;
d1 die Distanz von dem ersten Detektor zu dem Eintrittspunkt ist, wobei der Eintrittspunkt als der Punkt definiert ist, an dem der Strahl energiereicher Teilchen in das Ziel eintritt;
d2 der Abstand von dem zweiten Detektor zu dem Eintrittspunkt ist;
α der Winkel zwischen der Strahlrichtung und der Richtung, die von dem Eintrittspunkt zu dem ersten Detektor hin verläuft, ist;
β der Winkel zwischen der Strahlrichtung und der Richtung, die von dem Eintrittspunkt zu dem zweiten Detektor hin verläuft, ist.

11. Vorrichtung nach einem der vorstehenden Ansprüche 10 bis 11, wobei der zweite Gammadetektor in Bezug auf den ersten Gammadetektor derart lokalisiert ist, dass der erste und der zweite Gammadetektor prompte Gammas erfassen, die in Winkeln in Bezug auf die Strahlrichtung emittiert werden, die sich um mindestens 20° unterscheiden.

12. Vorrichtung nach einem der vorstehenden Ansprüche 9 bis 11, wobei der erste Gammadetektor zum Messen von prompten Gammas lokalisiert ist, die in einem Winkel von gleich oder größer als 100° in Bezug auf die Strahlrichtung emittiert werden, und wobei der zweite Gammadetektor zum Messen von prompten Gammas lokalisiert ist, die in einem Winkel von gleich oder kleiner als 80° in Bezug auf die Strahlrichtung emittiert werden.

**Revendications**

1. Procédé de vérification de la plage d'un rayonnement de particules hadroniques chargées (3) d'un dispositif de rayonnement (1) pour une radiothérapie en mesurant le rayon gamma instantané produit par le rayonnement de particules hadroniques chargées (3) lorsqu'il traverse le tissu d'un objet à irradier avec au moins un détecteur (2) étant capable de détecter des particules gamma individuelles (4) du rayon gamma instantané et au moins un analyseur (7), comprenant les étapes suivantes :

- lors de la détection d'une particule gamma (4), également appelée événement, un signal est créé dans le détecteur (2), moyennant quoi le signal est corrélé dans le temps avec l'arrivée de la particule gamma (4) dans le détecteur (2),

- l'analyseur (7), analysant le signal du détecteur (2), attribue soit à chaque événement, soit à des événements sélectionnés, un temps de détection,

- le dispositif de rayonnement (1) ou un détecteur de particules séparé (8) fournit un signal de référence, qui est corrélé à l'émersion de particules individuelles ou de groupes de particules depuis le dispositif de rayonnement (1) hors du dispositif de rayonnement (1) avec une incertitude temporelle égale ou inférieure à 10 ns,

- un temps t1 est obtenu à partir du signal de référence pour chaque événement ou chacun des événements sélectionnés dans l'analyseur (7), auquel la particule ou le groupe de particules, qui a produit la particule gamma (4) détectée par le détecteur (2), en outre décrite comme particule ou groupe de particules déclenchant l'événement, a traversé un plan de référence perpendiculaire à la direction du faisceau du dispostiif de rayonnement (1),

- un temps t2 est obtenu du détecteur (2), correspondant à l'impact de la particule gamma (4) dans le détecteur (2)

- la différence temporelle t entre le temps t2 de la détection de la particule gamma (4) dans le détecteur (2) et le temps t1 du passage du plan de référence par la particule ou le groupe de particules déclenchant l'événement est calculée pour chaque événement ou les événements sélectionnés dans l'analyseur (7), ladite différence temporelle étant une mesure de la longueur de la trajectoire de vol du rayonnement de particules hadroniques chargées (3),

- au moins une répartition statistique (6) des différences temporelles t est obtenue dans l'analyseur (7), décrite en outre comme répartition temporelle (6),

**caractérisé en ce que** le procédé comprend en outre les étapes suivantes :

- des paramètres intégraux sont déterminés à partir de la répartition temporelle (6), dans lequel les paramètres intégraux représentent des informations sur la plage du rayonnement de particules hadroniques chargées (3) dans l'objet à irradier, dans lequel les paramètres intégraux sont comparés à une répartition temporelle d'une référence pour déterminer des mouvements dans la plage du rayonnement de particules hadroniques chargées dans l'objet à irradier, de sorte qu'une déclaration par rapport à la conformité de la radiothérapie à vérifier avec l'appareil avec le plan de thérapie puisse être obtenue principalement à partir de cette répartition temporelle (6), et que

- l'analyseur (7) analyse la répartition temporelle (6), ce qui permet de dériver des informations sur la plage du faisceau de particules (3), de sorte qu'une déclaration relative à la conformité avec la thérapie à vérifier par le dispositif avec le plan de radiation soit donnée en temps réel, ladite déclaration étant dérivée d'un paramètre intégral de la répartition temporelle (6).

2. Procédé selon la revendication 1, **caractérisé en ce que** l'analyseur (7) sélectionne des événements, à partir desquels la répartition temporelle (6) est créée selon des critères prédéterminés par rapport au signal du détecteur (2), ces critères prédéterminés étant de préférence choisis dans un groupe comprenant

- l'intensité,
- l'amplitude, et/ou
- la forme du signal de temporisation du détecteur (2).

3. Procédé selon au moins l'une des revendications 1 à 2,
**caractérisé en ce que** le dispositif de rayonnement comprend au moins un second détecteur gamma pour détecter les gammas instantanés émis depuis ladite cible, le second détecteur étant situé par rapport au premier détecteur de telle sorte que le premier et le second détecteur détectent les gammas instantanés émis sous des angles différents par rapport à ladite direction de faisceau, obtenir une première répartition temporelle (6) pour le premier détecteur gamma et obtenir une seconde répartition temporelle (6) pour le second détecteur gamma, déterminer à partir de ladite première répartition temporelle une première largeur temporelle $\Delta T1$, déterminer à partir de ladite seconde répartition temporelle une seconde largeur temporelle $\Delta T2$, déterminer un décalage de déplacement de photon $\Delta DP$, défini comme la distance parcourue par un photon dans l'intervalle de temps égale à la différence entre la première et la seconde largeur temporelle multipliée par la vitesse de la lumière c : $\Delta DP = (\Delta T2 - \Delta T1)*c$, et déterminer la plage du faisceau de particules en corrélant ledit décalage de déplacement de photons $\Delta DP$ avec la différence d'emplacement du détecteur entre le premier et le second détecteur.

4. Procédé selon la revendication 3 selon lequel l'étape de détermination de ladite profondeur de pénétration comprend une étape de calcul d'une distance R dans la cible en résolvant l'équation suivante

$$\Delta DP = SQRT(R*R + d1*d1 - 2*R*d1*cos(\alpha)) -$$

$$SQRT(R*R + d2*d2 - 2*R*d2*cos(\beta)) - (d1 - d2),$$

moyennant quoi

> $\Delta DP$ est ledit décalage de déplacement de photon ;
> d1 est la distance entre le premier détecteur et le point d'entrée, le point d'entrée étant défini comme le point où le faisceau de particules énergétiques pénètre dans la cible ;
> d2 est la distance entre le second détecteur et le point d'entrée ;
> $\alpha$ est l'angle entre la direction de faisceau et la direction allant du point d'entrée au premier détecteur ;
> $\beta$ est l'angle entre la direction de faisceau et la direction allant du point d'entrée au second détecteur.

5. Procédé selon l'une quelconque des revendications 3 ou 4 dans lequel ledit second détecteur gamma est situé par rapport audit premier détecteur de telle sorte que le premier et le second détecteur détectent des gammas instantanés émis à des angles par rapport à la direction de faisceau (6) qui diffèrent d'au moins 20°.

6. Procédé selon l'une quelconque des revendications 3 ou 4 dans lequel ledit premier détecteur gamma est situé pour mesurer des gammas instantanés émis à un angle égal ou supérieur à 100° par rapport à la direction de faisceau et ledit second détecteur gamma est situé pour mesurer des gammas instantanés émis à un angle égal ou inférieur à 80° par rapport à la direction de faisceau.

7. Appareil permettant de vérifier la plage du rayonnement de particules hadroniques chargées (3) provenant d'un dispositif de rayonnement (1) pour une radiothérapie, mesurant le rayon gamma instantané produit par le rayonnement de particules hadroniques chargées lorsqu'il traverse le tissu d'un objet à irradier, configuré pour exécuter un procédé selon l'une des revendications 1 à 6 avec au moins un premier détecteur (2), défini pour détecter des particules gamma individuelles du rayon gamma instantané et au moins un analyseur (7), le détecteur (2) étant un détecteur générateur de signal (2) défini pour générer un signal après la détection d'une particule gamma (4), ladite détection étant en outre décrite comme un événement, ledit signal étant corrélé à l'arrivée de la particule gamma (4) au niveau du détecteur (2), moyennant quoi le détecteur (2) est connecté à l'analyseur (7), moyennant quoi l'analyseur (7) est configuré pour analyser le signal provenant du détecteur (2) et pour attribuer soit à chaque événement, soit à des événements sélectionnés, le temps de sa/leur détection, moyennant quoi l'analyseur (7) est en outre connecté au dispositif de rayonnement (1) ou à un détecteur de particules séparé (8), moyennant quoi le dispositif de rayonnement (1) ou le détecteur de particules (8) est configuré pour fournir un signal de référence, qui est corrélé à l'émission de particules individuelles ou de groupes de particules depuis le dispositif de rayonnement (1) hors du dispositif de rayonnement (1), et que l'analyseur (7) est un analyseur (7),

> - configuré pour déterminer un temps à partir du signal de référence pour soit chaque événement, soit des événements sélectionnés, auquel la particule ou le groupe de particules, qui a créé la particule gamma (4) étant détectée dans le détecteur (2), référencée comme particule ou groupe de particules déclenchant l'événement. a traversé un plan de référence étant perpendiculaire à la sortie de faisceau du dispositif de rayonnement (1),
> - est configuré pour calculer une différence temporelle $\Delta t1$ entre le temps de la détection de la particule gamma (4) dans le détecteur (2) et le temps de la traversée du plan de référence par la particule ou le groupe de particules définissant l'événement pour chaque événement ou les événements sélectionnés, ladite différence temporelle étant une mesure de la longueur de la trajectoire de vol du rayonnement de particules hadroniques chargées (3),
> - est configuré pour dériver au moins une répartition statistique (6) des différences temporelles, appelée répartition temporelle (6),
> **caractérisé en ce que** l'analyseur est en outre configuré
> - pour déterminer des paramètres intégraux à partir de la répartition temporelle (6), dans lequel les paramètres intégraux représentent des informations sur la plage du faisceau de particules hadroniques chargées (3) et pour comparer les paramètres intégraux à une répartition temporelle d'une référence et configuré pour déterminer des mouvements dans la plage du rayonnement de particules hadroniques chargées dans l'objet à irradier,

ce qui permet d'obtenir une déclaration concernant la conformité de la thérapie, vérifiée avec l'appareil, avec le plan d'irradiation principalement basé sur cette répartition temporelle (6).

8. Appareil selon la revendication 7, **caractérisé en ce que** le détecteur (2), capable de détecter des particules gamma individuelles (4), est

- un détecteur à scintillation (2), comprenant au moins un scintillateur et au moins un photodétecteur,
- un détecteur Tcherenkov (2), comprenant au moins un photodétecteur, couplé à un milieu transparent, ou une
- chambre à plaques résistives (RPC) (2).

9. Appareil selon la revendication 7, **caractérisé en ce que** ledit appareil comprend en outre un second détecteur gamma, configuré pour détecter des gammas instantanés émis depuis ladite cible, étant situé par rapport audit premier détecteur de telle sorte que le premier et le second détecteur détectent des particules gamma instantanées émises sous des angles différents par rapport à la direction de faisceau, l'analyseur calculant les différences temporelles $\Delta t1$ et $\Delta t2$ pour les deux détecteurs de manière à obtenir des répartitions temporelles pour les deux détecteurs, comprenant en outre un analyseur comprenant un algorithme configuré pour déterminer à partir de ladite première différence temporelle $\Delta t1$ une première largeur temporelle $\Delta T1$ et à partir de ladite seconde différence temporelle $\Delta t2$ une seconde largeur temporelle $\Delta T2$, déterminant en outre un décalage de déplacement de photon $\Delta DP$ défini comme la distance parcourue par un photon dans l'intervalle de temps égale à la différence entre la première et la seconde largeur temporelle : $\Delta DP = (\Delta T2 - \Delta T1)*c$, moyennant quoi c est égal à la vitesse de la lumière, déterminer une profondeur de pénétration du faisceau de particules en corrélant ledit décalage de déplacement de photon $\Delta DP$ avec la différence d'emplacement du détecteur entre le premier et le second détecteur.

10. Appareil selon la revendication précédente, **caractérisé en ce que** l'analyseur comprend des moyens de calcul configurés pour calculer une distance R dans la cible en résolvant l'équation

$$\Delta DP = \quad SQRT(R*R + d1*d1 - 2*R*d1*cos(\alpha)) -$$

$$SQRT(R*R + d2*d2 - 2*R*d2*cos(\beta)) - (d1 - d2),$$

moyennant quoi

$\Delta DP$ est ledit décalage de déplacement de photon ;
d1 est la distance entre le premier détecteur et le point d'entrée, le point d'entrée étant défini comme le point où le faisceau de particules énergétiques pénètre dans la cible ;
d2 est la distance entre le second détecteur et le point d'entrée ;
$\alpha$ est l'angle entre la direction de faisceau et la direction allant du point d'entrée au premier détecteur ;
$\beta$ est l'angle entre la direction de faisceau et la direction allant du point d'entrée au second détecteur.

11. Appareil selon l'une quelconque des revendications 10 ou 11 précédentes dans lequel ledit second détecteur gamma est situé par rapport audit premier détecteur de telle sorte que le premier et le second détecteur gamma détectent des gammas instantanés émis à des angles par rapport à la direction de faisceau qui diffèrent d'au moins 20°.

12. Appareil selon l'une quelconque des revendications 9 à 11 dans lequel ledit premier détecteur gamma est situé pour mesurer des gammas instantanés émis à un angle égal ou supérieur à 100° par rapport à la direction de faisceau et ledit second détecteur gamma est situé pour mesurer des gammas instantanés émis à un angle égal ou inférieur à 80° par rapport à la direction de faisceau.

Fig. 1

Fig. 2

EP 3 047 309 B1

Fig. 3

EP 3 047 309 B1

Fig.4

EP 3 047 309 B1

Fig. 5

Fig. 6

Fig. 7

Fig. 8

Fig. 9

EP 3 047 309 B1

Fig. 10

EP 3 047 309 B1

Fig. 11

A: $\alpha = 180°$, $\beta = 0°$, S = 6,67 ps/mm
B: $\alpha = 135°$, $\beta = 45°$, S = 4,58 ps/mm
C: $\alpha = 120°$, $\beta = 60°$, S = 3,34 ps/mm
D: $\alpha = 110°$, $\beta = 70°$, S = 2,44 ps/mm
E: $\alpha = 100°$, $\beta = 80°$, S = 1,53 ps/mm

$\Delta T2 - \Delta T1$ (ns)

D (mm)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20110057110 A1
- US 20120025076 A1

**Non-patent literature cited in the description**

- Proton Therapy Physics. CRC Press, Taylor & Francis Group, LLC, 2012, 512-514
- **TESTA et al.** *Nucl.Inst.Meth. B*, 2009, vol. 267, 993
- **TESTA**. Charged particle therapy, ion range verification, prompt radiation. Physics Universite Claude Bernard, 2010
- **G.F. KNOLL**. Radiation Detection and Measurement. John Wiley & Sons, Inc., 2010, 733-735
- **G.F. KNOLL**. Radiation Detection and Measurement. John Wiley & Sons, Inc., 2010, 201-203
- **GUETH et al.** *Phys. Med. Biol.*, 2013, vol. 58, 4563-4577
- **SCHAART et al.** *Phys. Med. Biol.*, 2010, vol. 55, N179-N189